# EUROPEAN PATENT APPLICATION

(11) **EP 4 086 342 A1**
(43) Date of publication of application: **09.11.2022**
(21) Application number: 20911252.3
(22) Date of filing: 29.12.2020
(51) Int. Cl.: C12N 5/10, A61K 35/17, A61P 35/00, A61P 31/00, A61P 37/06, A61P 37/08, A61P 37/02, C12N 15/85

(54) **MODIFIED IMMUNE EFFECTOR CELL AND PREPARATION METHOD THEREFOR**

(30) Priority: 02.01.2020 CN 202010002929
(71) Applicant: Ningbo T-MAXIMUM Biopharmaceuticals Co., Ltd., Ningbo, Zhejiang (CN)
(72) Inventor: SHANG, Xiaoyun, Suzhou, Jiangsu 215000 (CN); JIANG, Haijuan, Suzhou, Jiangsu 215000 (CN); WANG, Dan, Suzhou, Jiangsu 215000 (CN); SHEN, Hui, Suzhou, Jiangsu 215000 (CN); MA, Li, Suzhou, Jiangsu 215000 (CN); XIN, Yu, Suzhou, Jiangsu 215000 (CN); XU, Fanli, Suzhou, Jiangsu 215000 (CN); LI, Jialu, Suzhou, Jiangsu 215000 (CN); MA, Shaowen, Suzhou, Jiangsu 215000 (CN); ZHAO, Dan, Suzhou, Jiangsu 215000 (CN)
(74) Representative: Böhm, Brigitte
(86) International application number: PCT/CN2020/140799
(87) International publication number: WO 2021/136263

(57) **Abstract**

Provided is a modified immune effector cell, wherein compared with the expression and/or activity of the corresponding gene in a corresponding unmodified cell, the expression and/or activity of the TRAC gene and the HLA-A gene are down-regulated, the expression and/or activity of the B2M gene is not down-regulated, and the expression and/or activity of the CIITA gene is not down-regulated. Also provided is a method for preparing the modified immune effector cell.

## Description

### Technical Field

The present application relates to the biomedical field, in particular to a modified immunologic effector cell and a preparation method thereof.

### BACKGROUND

Anti-tumor immunotherapies can cause long-term and strong response in a variety of malignant tumors, can be used to treat many different types of cancers, and exhibit a broad potential. Currently, anti-tumor immunotherapies mainly include two types of immunotherapies: immunocyte targeted monoclonal antibody (mAb) therapy and adoptive cell therapy (ACT). Of those, ACT refers to a therapy relying on returning autologous or allogeneic lymphocytes which are stimulated and amplified *in vitro* back into the human body so as to achieve an anti-tumor effect, but such a therapy only produces a relatively good effect in patients with MHC polymorphism consistency. The CAR-T is a new and effective MHC-independent adoptive cell therapy. CAR, also known as chimeric antigen receptor, is an artificial receptor that mimics the function of TCR. It can specifically recognize an antigen on the surface of tumor cells, so as to target and kill the tumor cells. However, timely and successful production and infusion of autologous CAR T cells is the biggest obstacle to implement an effective CAR T cell therapy. For example, if patients were previously treated with a chemotherapy regimen, the chemotherapy drugs can result in difficult expansion or decreased function of the T cells of the patients *in vitro,* resulting in insufficient or poor-quality CAR-T cell products. Moreover, autologous CAR-T is commonly used in ALL or CLL patients at present, and its application in solid tumor patients is facing great challenges. For example, the heterogeneity of tumor antigen limits the application of autologous CAR-T. In order to increase the flexibility of CAR and broaden the scope of antigen recognition, universal CAR-T emerges. The main design principle of universal CAR-T cells is to produce tumor antigen-specific T cells from allogeneic healthy donors. The so-obtained CAR-T cells exhibit increased amplification efficiency and activity. However, the endogenous TCR of allogeneic T cells can recognize the allogeneic antigen of receptor, which will lead to a graftversus-host disease (GVHD). At the same time, the expression of HLA on the surface of allogeneic T cells will lead to a rapid rejection of host immune system (HVGR). Therefore, the problem of immune rejection response caused by allogeneic cell therapy needs to be further addressed.

### SUMMARY OF THE INVENTION

The present application provides a modified immunologic effector cell, wherein the expression and/or activity of a TRAC gene and a HLA-A gene are/is down-regulated, the expression and/or activity of a B2M gene are/is not down-regulated, and the expression and/or activity of a CIITA gene are/is not down-regulated, as compared with the expression and/or activity of the corresponding gene in the corresponding cell which is not modified.

In some embodiments, the modification results in down-regulation of expression and/or activity of two genes, wherein the two genes consist of the TRAC gene and the HLA-A gene.

In some embodiments, the expression and/or activity of the TRAC gene and the HLA-A gene are/is down-regulated, the expression and/or activity of the B2M gene are/is not down-regulated, and the expression and/or activity of the CIITA gene are/is not down-regulated, as compared with the corresponding wild type cell.

In some embodiments, as compared with the corresponding wild type cell, the expression and/or activity of the two genes are/is down-regulated, wherein the two genes consist of the TRAC gene and the HLA-A gene.

In some embodiments, the immunologic effector cell includes T cell.

In some embodiments, the down-regulation of the expression level and/or activity of the genes includes a down-regulation of expression and/or activity of a nucleic acid molecule encoding the genes; and/or a down-regulation of expression and/or activity of a protein product encoded by the genes.

In some embodiments, the modification includes gene mutation and/or gene silencing.

In some embodiments, the modification includes administering one or more substances selected from the group consisting of antisense RNA, siRNA, shRNA, and CRISPR/Cas9 system to the immunologic effector cell.

In some embodiments, the modification includes administering CRISPR/Cas9 system to the immunologic effector cell.

In some embodiments, the allele of the HLA-A gene is selected from the group consisting of A^{∗}02, A^{∗}11, A^{∗}24, A^{∗}30, A^{∗}33, A^{∗}03, A^{∗}01 and A^{∗}26.

In some embodiments, the expression level and/or activity of at most 2 alleles of the HLA-A gene are/is down-regulated.

In some embodiments, the expression level and/or activity of 1 allele of the HLA-A gene are/is down-regulated.

In some embodiments, the modification includes administering an sgRNA targeting an exon of the HLA-A gene to the immunologic effector cell.

In some embodiments, the sgRNA targeting the exon of the HLA-A gene includes a nucleotide sequence shown in any one of SEQ ID No. 16-54 and 91-92.

In some embodiments, the modification further includes administering an sgRNA targeting an exon of the TRAC gene to the immunologic effector cell.

In some embodiments, the sgRNA targeting the exon of the TRAC gene includes a nucleotide sequence shown in any one of SEQ ID No. 1-15.

In some embodiments, the antisense RNA includes a nucleotide sequence as shown in any one of SEQ ID No. 93-96.

In some embodiments, the modification further includes administering Cas protein to the cell.

In some embodiments, the Cas protein includes a Cas9 protein.

In some embodiments, the immunologic effector cells include a nucleic acid encoding a chimeric antigen receptor (CAR), and the CAR includes an antigen binding domain, a hinge region, a transmembrane domain, a costimulatory structure, and a primary signal transduction domain.

In some embodiments, the antigen binding domain specifically binds to a tumor antigen.

In some embodiments, the tumor antigen is selected from the group consisting of: CD19, CD133, CD123, CD22, CD30, CD171, CA125, C-met, L1CAM, EC, DLL3, CD99, CS1, 5T4, CD138, CS-1 (also known as CD2 subclass 1, CRACC, SLAMF7, CD319 or 19A24), C-type lectin-like molecule-1 (CLL-1 or CLECL1), CD33, epidermal growth factor receptor variant III (EGFRvIII), ganglioside G2 (GD2), ganglioside GD3, TNF receptor family member B cell mature antigen (BCMA), Tn antigen (e.g., Tn Ag, GalNAcα-Ser/Thr), prostate-specific membrane antigen (PSMA); receptor tyrosine kinase-like orphan receptor 1 (ROR1), Fms-like tyrosine kinase 3 (FLT3); tumor-associated glycoprotein 72 (TAG72), CD38, CD44v6, carcino-embryonic antigen (CEA), epithelial cell adhesion molecule (EPCAM), B7H3 (CD276), KIT (CD117), interleukin-13 receptor subunit α-2 (IL-13Ra2 or CD213A2), mesothelin, interleukin 11 receptor α (IL-11Ra), prostate stem cell antigen (PSCA), protease serine 21, vascular endothelial growth factor receptor 2 (VEGFR2), Lewis (Y) antigen, CD24, platelet-derived growth factor receptor β (PDGFR-β), stage-specific embryonic antigen-4 (SSEA-4), CD20, folate receptor α, receptor tyrosine-protein kinase ERBB2 (Her2/neu), cell surface-associated mucoprotein 1 (MUC1), epidermal growth factor receptor (EGFR), neurocyte adhesion molecule (NCAM), Prostase, prostate acid phosphatase (PAP), elongation factor 2 mutant (ELF2M), ephrin B2, fibroblast activated protein α (FAP), insulin-like growth factor 1 receptor (IGF-I receptor), carbonic anhydrase IX (CAIX), proteasome (e.g., proteasome, giantin factor) subunit B-type 9 (LMP2), glycoprotein 100 (gp100), oncogene fusion protein (bcr-abl) composed of breakpoint cluster region (BCR) and Abelson murine leukemia virus oncogene homolog 1 (Abl), tyrosinase, ephrin A-type receptor 2 (EphA2), fucosyl GM1; sialyl Lewis adhesion molecule (sLe), transglutaminase 5 (TGS5), high molecular weight-melanoma-associated antigen (HMWMAA), o-acetyl-GD2 ganglioside (OAcGD2), folate receptor β, tumor endothelial marker 1 (TEM1/CD248), tumor endothelial marker 7-associated (TEM7R), tight junction protein 6 (CLDN6), thyrotropin receptor (TSHR), G-protein-coupled receptor C-class Group 5 Member D (GPRC5D), chromosome X ORF 61 (CXORF61), CD97, CD179a, anaplastic lymphoma kinase (ALK), polysialic acid, placental specificity 1 (PLAC1), hexose moiety of globoH glycoceramide (GloboH), mammary gland differentiation antigen (NY-BR-1), uroplakin 2 (UPK2), hepatitis virus A cell receptor 1 (HAVCR1), adrenaline receptor β3 (ADRB3), pannexin 3 (PANX3), G-protein-coupled receptor 20 (GPR20), lymphocyte antigen 6 complex loci K9 (LY6K), olfactory receptor 51E2 (OR51E2), TCRγ variable ORF protein (TARP), Wilm tumor protein (WT1); cancer/testis antigen 1 (NY-ESO-1), cancer/testis antigen 2 (LAGE-1a), melanoma-associated antigen 1 (MAGE-A1), ETS translocation variant gene 6 located on chromosome 12p (ETV6-AML), sperm protein 17 (SPA17), X antigen family member 1A (XAGE1), angiogenin cell surface receptor 2 (Tie 2), melanoma cancer/testis antigen-1 (MAD-CT-1), melanoma cancer/testis antigen-2 (MAD-CT-2), Fos-associated antigen 1, p53, p53 mutant, prostate-specific protein (prostein), prostate cancer tumor antigen-1 (PCTA-1 or galectin 8), T cell-recognized melanoma antigen 1 (MelanA or MARTI); rat sarcoma (Ras) mutant, human telomerase reverse transcriptase (hTERT), sarcoma translocation breakpoint, melanoma anti-apoptosis protein (ML-IAP), ERG (transmembrane protease, serine 2 (TMPRSS2) ETS fusion gene), N-acetylglucosaminyltransferase V (NA17), paired frame protein Pax-3 (PAX3), androgen receptor, cyclin B1, v-myc avian myelocytosis virus oncogene neuroblastoma-derived homologs (MYCN), Ras homolog family member C (RhoC), tyrosinase-associated protein 2 (TRP-2), cytochrome P450 1B 1 (CYP1B1), T cell-recognized squamous cell cancer antigen 3 (SART3), paired frame protein Pax-5 (PAX5), proacrosomal protein binding protein sp32 (OY-TES1), lymphocyte-specific protein tyrosine kinase (LCK), A kinase anchorin 4 (AKAP-4), synovial sarcoma X breakpoint 2 (SSX2), receptor of advanced glycation end products (RAGE-1), legumain, human papilloma virus E6 (HPV E6), human papilloma virus E7 (HPV E7), intestinal carboxylesterase, mutated heat shock protein 70-2 (mut hsp70-2), CD79a, CD79b, CD72, leukocyte-associated immunoglobulin-like receptor 1 (LAIR1), Fc fragment of IgA receptor (FCAR or CD890), leukocyte immunoglobin-like receptor superfamily A member 2 (LILRA2), CD300 molecule-like family member f (CD300LF), C-type lectin domain family 12 member A (CLEC12A), bone marrow stromal cell antigen 2 (BST2), EGF-like modulus-containing mucoprotein-like hormone receptor-like 2 (EMR2), lymphocyte antigen 75 (LY75), glypican-3 (GPC3), Fc receptor-like 5 (FCRL5) and/or immunoglobulin λ-like peptide 1.

In some embodiments, the antigen binding domain is selected from the group consisting of a monoclonal antibody, a polyclonal antibody, a human antibody, a humanized antibody, a single domain antibody, and an antigen binding fragment.

In some embodiments, the antigen binding domain targets a solid tumor.

In some embodiments, the solid tumor is selected from the group consisting of: lung cancer, breast cancer, colon cancer, renal cell carcinoma, liver cancer, non-small cell lung cancer, small intestine cancer, esophagus cancer, osteosarcoma, pancreatic cancer, skin cancer, head or neck cancer, cutaneous or intraocular malignant melanoma, uterus cancer, ovarian cancer, rectal cancer, anal cancer, stomach cancer, testis cancer, fallopian tube carcinoma, endometrial cancer, cervical cancer, vaginal cancer, vulval cancer, Hodgkin's disease, non-Hodgkin's lymphoma, carcinoma of endocrine system, thyroid cancer, parathyroid cancer, adrenal carcinoma, soft tissue sarcoma, urethral carcinoma, carcinoma of penis, pediatric solid tumor, bladder cancer, renal or ureteral cancer, carcinoma of renal pelvis, central nervous system (CNS) tumor, primary CNS lymphoma, tumor angiogenesis, spinal tumor, brainstem glioma, pituitary adenoma, Kaposi sarcoma, epidermoid, squamous cell carcinoma, and T cell lymphoma.

In some embodiments, the antigen binding domain targets a non-solid tumor.

In some embodiments, the non-solid tumor is selected from the group consisting of chronic lymphoblastic leukemia (CLL), acute leukemia, acute lymphoblastic leukemia (ALL), B cell acute lymphoblastic leukemia (B-ALL), T cell acute lymphoblastic leukemia (T-ALL), chronic myeloid leukemia (CML), acute myeloid leukemia (AML), B cell prolymphocytic leukemia, blast cell plasmacytoid dendritic cytoma, Burkitt's lymphoma, diffuse large B cell lymphoma, follicular lymphoma, hairy cell leukemia, small or large cell follicular lymphoma, malignant lymphoproliferative conditions, MALT lymphoma, mantle cell lymphoma, marginal zone lymphoma, multiple myeloma, myelodysplasia and myelodysplastic syndrome, Hodgkin's lymphoma, plasmablast lymphoma, Plasmacytoid dendritic cytoma, B lymphocytoma, and Waldenstrom macroglobulinemia.

In some embodiments, the transmembrane domain includes one protein derived from a protein selected from the group consisting of CD28, CD3e, CD27, CD3ε, CD45, CD4, CD5, CD8, CD9, CD16, CD22, CD33, CD37, CD64, CD80, CD86, CD134, CD137, CD154, CD19, IL2Rβ, IL2Rγ, IL7 Rα, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2.

In some embodiments, the costimulatory domain includes a costimulatory domain of protein selected from the group consisting of: CD137, CD28, CD27, OX40, CD30, CD40, PD-1, ICOS, LFA-1, CD2, CD7, CD160(BY55), LIGHT, NKG2C, B7-H3, CDS, ICAM-1, GITR, BAFFR, HVEM(LIGHTR), SLAMF7, NKp80(KLRF1), CD19, CD4, CD8α, CD8β, IL2Rβ, IL2Rγ, IL7 Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1(CD226), SLAMF4(CD244, 2B4), CD84, CD96(Tactile), CEACAM1, CRTAM, Ly9(CD229), CD160, PSGL1, CD100(SEMA4D), CD69, SLAMF6(NTB-A, Ly108), SLAM(SLAMF1, CD150, IPO-3), BLAME(SLAMF8), SELPLG(CD162), LTBR, LAT, GADS, SLP-76, PAG/Cbp, NKp44, NKp30, NKp46 and/or NKG2D.

In some embodiments, the primary intracellular signal transduction domain includes a functional signal transduction domain of protein selected from the group consisting of CD3ζ, FcRγ(FCER1G), FcyRIIa, FcRβ(FcεR1b), CD3y, CD3δ, CD3ε, CD79a, CD79b, DAP10 and/or DAP12.

In some embodiments, the hinge region connects the antigen binding domain with the transmembrane domain, and includes a hinge region of protein selected from the group consisting of human Ig (immunoglobulin) hinge region, a GS linker, a KIR2DS2 hinge region, or a CD8a hinge region.

The present application further provides a method of preparing the modified immunologic effector cell of the present application, including the following steps: as compared with the expression and/or activity of the corresponding genes in corresponding cells which are not modified, down-regulating the expression and/or activity of the TRAC gene and the HLA-A gene in the immunologic effector cell, not down-regulating the expression and/or activity of the B2M gene, and not down-regulating the expression and/or activity of the CIITA gene.

In some embodiments, the modification results in down-regulation of expression and/or activity of two genes, wherein the two genes consist of the TRAC gene and the HLA-A gene.

In some embodiments, the expression and/or activity of the TRAC gene and the HLA-A gene are/is down-regulated, the expression and/or activity of the B2M gene are/is not down-regulated, and the expression and/or activity of the CIITA gene are/is not down-regulated, as compared with the corresponding wild type cell.

In some embodiments, as compared with the corresponding wild type cell, the expression and/or activity of the two genes are down-regulated, wherein the two genes consist of the TRAC gene and the HLA-A gene.

In some embodiments, the down-regulation of the expression level and/or activity of the genes include a down-regulation of expression and/or activity of a nucleic acid molecule encoding the genes; and/or a down-regulation of expression and/or activity of a protein product encoded by the genes.

In some embodiments, the modification includes gene mutation and/or gene silencing.

In some embodiments, the modification includes administering one or more substances selected from the group consisting of: antisense RNA, siRNA, shRNA, and CRISPR/Cas9 system to the immunologic effector cell.

In some embodiments, the modification includes administering CRISPR/Cas9 system to the immunologic effector cell.

In some embodiments, the modification includes administering an sgRNA targeting an exon of the HLA-A gene to the immunologic effector cell.

In some embodiments, the sgRNA targeting the exon of the HLA-A gene includes a nucleotide sequence shown in any one of SEQ ID No. 16-54 and 91-92.

In some embodiments, the modification includes administering an sgRNA targeting an exon of the TRAC gene to the immunologic effector cell.

In some embodiments, the sgRNA targeting the exon of the TRAC gene includes a nucleotide sequence shown in any one of SEQ ID No. 1-15.

In some embodiments, the antisense RNA includes a nucleotide sequence as shown in any one of SEQ ID No. 93-96.

In some embodiments, the modification further includes administering Cas protein to the cell.

In some embodiments, the Cas protein includes Cas9 protein.

The present application further provides a composition including the immunologic effector cells of the present application and a pharmaceutically acceptable carrier.

In some embodiments, the composition includes a cell population, wherein the cell population includes the modified immunologic effector cell of the present application.

The present application further provides use of the modified immunologic effector cells of the present application for preparing a CAR-T cell.

The present application further provides use of the modified immunologic effector cells of the present application in manufacture of a drug for allogeneic therapy.

The present application further provides a method of allogeneic therapy, including administering the modified immunologic effector cell of the present application to a patient or subject.

The present application further provides use of the modified immunologic effector cell of the present application for allogeneic therapy.

The present application further provides use of the modified immunologic effector cell of the present application in manufacture of a drug for treating tumors.

The present application further provides a method of treating tumor, including administering the modified immunologic effector cell of the present application to a patient or subject.

The present application further provides the modified immunologic effector cell for treating a tumor.

In some embodiments, the tumor includes solid and non-solid tumors.

In some embodiments, the tumor is selected from the group consisting of: lung cancer, breast cancer, colon cancer, renal cell carcinoma, liver cancer, non-small cell lung cancer, small intestine cancer, esophagus cancer, osteosarcoma, pancreatic cancer, skin cancer, head or neck cancer, cutaneous or intraocular malignant melanoma, uterus cancer, ovarian cancer, rectal cancer, anal cancer, stomach cancer, testis cancer, fallopian tube carcinoma, endometrial cancer, cervical cancer, vaginal cancer, vulval cancer, Hodgkin's disease, non-Hodgkin's lymphoma, carcinoma of endocrine system, thyroid cancer, parathyroid cancer, adrenal carcinoma, soft tissue sarcoma, urethral carcinoma, carcinoma of penis, pediatric solid tumor, bladder cancer, renal or ureteral cancer, carcinoma of renal pelvis, central nervous system (CNS) tumor, primary CNS lymphoma, tumor angiogenesis, spinal tumor, brainstem glioma, pituitary adenoma, Kaposi sarcoma, epidermoid, squamous cell carcinoma, and T cell lymphoma.

Persons skilled in the art can easily recognize other aspects and advantages of the present application from the following detailed description.

The following detailed description only shows and describes exemplary embodiments of the present application. As persons skilled in the art will appreciate, the present application enables persons skilled in the art to make modifications to the disclosed embodiments without departing the spirit and scope of the invention involved in the present application. Correspondingly, the accompany drawings and description in the specification of the present application are only illustrative, rather than restrictive.

### BRIEF DESCRIPTION OF THE DRAWINGS

The specific features of the invention involved in the present application are shown in the appended claims. By referring to the exemplary embodiments as detailedly described below and the accompanying drawings, the features and advantages of the invention involved in the present application can be better understood. The accompany drawings are briefly described as follows:
FIG. 1 shows the Sanger sequencing results of the TRAC gene of the present application after Sg9RNA editing.
FIG. 2 shows the TA clone detection results of the TRAC gene of the present application after Sg9RNA editing.
FIG. 3 shows the flow cytometric results of the TRAC gene of the present application after Sg9RNA editing.
FIG. 4 shows the Sanger sequencing results of the HLA-A02 gene of the present application after Sg2RNA editing.
FIG. 5 shows the Sanger sequencing results of the HLA-A02 gene of the present application after Sg5RNA editing.
FIG. 6 shows the Sanger sequencing results of the HLA-A11 gene of the present application after Sg10-3RNA editing.
FIG. 7 shows the Sanger sequencing results of the HLA-A11 gene of the present application after Sg21RNA editing.
FIGs. 8A-8B show the results of simultaneous knockout of HLA-A02 and TRAC in the modified immune effector cells of the present application.
FIGs. 9A-9B show the protein levels of HLA-A02 and TRAC in the modified immune effector cells of the present application.
FIGs. 10A-10D show the mRNA levels of TRAC, HLA-A, B2M and CIITA in the modified immune effector cells of the present application.
FIGs. 11A-11B show the protein levels of B2M and CIITA in the modified immune effector cells of the present application.
FIGs. 12A-12D show the protein levels of TRAC, HLA-A, B2M and CIITA in the modified immune effector cells of the present application.
FIGs. 13A-13B show the knockout status of TRAC and HLA-A at mRNA levels in the modified immune effector cells of the present application.
FIGs. 14A-14B show the protein levels of CD69 and CD137 in the modified immune effector cells of the present application.
FIG. 15 shows the co-culture status of the modified immune effector cells of the present application and NK cells
FIG. 16 shows the level of IFN-γ expressed in the modified immune effector cells of the present application.
FIGs. 17A-17D show the protein levels of TRAC, HLA-A, B2M and CIITA in the modified immune effector cells of the present application.
FIG. 18 shows the efficiency of the modified immune effector cells of the present application for infecting the CAR.
FIG. 19 shows the amplification fold of the modified immune effector cells of the present application.
FIG. 20 shows the killing effect of the modified immune effector cells of the present application on CD19 positive target cells.
FIG. 21 shows a dosage regimen for administration of the modified immune effector cells of the present application.
FIG. 22 shows the killing effect of the modified immune effector cells of the present application on the tumor in mice.

### DETAILED DESCRIPTION OF THE EMBODIMENTS

Hereinafter the embodiments of the present application are described by reference to particular examples. Persons skilled in the art can readily understand other advantages and effects of the inventions of the present application from the disclosure of the present specification.

Hereinafter the present application will be further described. In the present invention, unless otherwise specified, the scientific and technical terms as used herein will have meanings commonly understood by persons skilled in the art. Moreover, all the terms and laboratory operation steps associated with protein and nucleic acid chemistry, molecular biology, cell and tissue culture, microbiology and immunology used herein are widely used terms and conventional steps in the corresponding fields. Meanwhile, in order to better understand the present invention, definitions and explanations of related terms are provided below.

In the present application, the term "immune effector cells" generally refers to immune cells that take part in the immune response and perform the effector function. For example, the performing effector function can include clearing a foreign antigen or promoting a response of immune effector, etc. Immune effector cells can include plasmacytes, T cells, B cells, natural killer (NK) cells, natural killer T (NKT) cells, mastocytes, and bone marrow-derived phagocytes.

In the present application, the term "modification/modified" generally refers to changing cell status or structure and/or change of cell status or structure. The change is usually in relation to the status or structure of the corresponding cells that are not modified. The change can include changes in the expression level or function of endogenous cell genes, e.g., down-regulating, upregulating the expression level of endogenous cell genes or not expressing the endogenous cell genes by means of genetic engineering. The genetic engineering means can include homologous recombination, CRISPR/Cas9 system gene editing, etc. The change can also include changes in cell protein expression, structure or function, e.g., changes in protein expression, structure or function realized by changing the expression level or function of corresponding endogenous genes, such as, changes in protein expression, structure or function realized by regulating protein translation and post-translational modification. The changes can also include introduction of foreign gene, expression of foreign protein and so on.

In the present application, the term "TRAC" generally refers to T cell receptor α chain constant region (T cell receptor alpha constant). T cell receptor (TCR) generally refers to a specific receptor located on the surface of T cell that can recognize an antigen binding to a major histocompatibility complex (MHC) molecule. TCR is commonly composed of two different protein chains (i.e., a heterogeneous dimer). In humans, the TCR in a majority of T cells is composed of one alpha-chain and a beta-chain (which are encoded by TRA and TRB, respectively), and such T cells are called α,β-T cells. In a minority of T cells, the TCR is composed of γ-chain and δ-chain (which are encoded by TRG and TRD, respectively), and such T cells are called γ,δ-T cells. Generally, α,β-T cells account for about 95% of the total T cells, while γ,δ-T cells account for about 5% of the total T cells. This ratio varies during the development of individuals and in diseased states, such as leukemia, and varies between species. Each chain of TCR contains variable regions and constant regions. In humans, the gene encoding the α chain (TRA, e.g., information as shown by HGNC: 12027) is located on chromosome 14 and consists of multiple gene fragments, including a variable fragment (V), a junction fragment (J) and a constant region (C). The TRAC gene generally refers to a gene sequence encoding the constant region (C) of α chain of T cell receptor (e.g., information as shown by HGNC: 12029), which is located on chromosome 14 (14q11.2; 14: 22,547,505-22,552,131). Generally, one of the genes of the variable fragments (V) encoding the N-fragment of antigen recognition domain is rearranged with one of the junction fragments (J) to produce a functional V-region exon, which is transcribed and linked to the constant region (C) by splicing, thereby forming a coding sequence of α chain of the T cell receptor.

In the present application, "HLA-A" generally refers to a class of polypeptide chains of human leukocyte antigens which are encoded by the HLA-A gene located at human chromosome 6p21.3 (e.g., information as shown by HGNC:4931). HLA-A is one of the three major types of polypeptides that constitute MHC Class I molecules on the surface of human cells, and others further include HLA-B and HLA-C. A heterodimer composed of an α chain encoded by HLA-A gene and a β chain encoded by B2M gene (β2-microglobulin) is an HLA-A class MHC I molecule. The α chain encoded by HLA-A gene can include an α1 domain, an α2 domain, an α3 domain, a transmembrane region, and a cytoplasmic region, wherein the α1 domain, and the α2 domain can be combined with a peptide fragment so as to present the peptide fragment to the immune cells by the MHC I molecules (e.g., HLA-A class). In humans, similar to most mammals, the α chain of MHC I molecule is polymorphic, and there are many variations in the primary structure thereof. By December 2013, there are 2432 known HLA-A alleles in total, which encode 1740 active proteins and 117 inactive proteins. In the present application, HLA-A alleles can include sequence information of different HLA-A alleles included in the IMGT/HLA Database Version 3.38.0 (https://www.ebi.ac.uk/ipd/imgt/hla/) and named by the WHO HLA Factor Nomenclature Committee.

In the present application, the term "B2M" generally refers to β2-microglobulin that is one of the components of the MHC Class I molecule. β2 microglobulin (also known as β chain) can form an MHC Class I molecule, together with an α chain encoded by HLA. B2M is generally expressed in all karyocytes. In humans, β2 microglobulin is encoded by the B2M gene located at 15q21.1 (e.g., information as shown by HGNC:914).

In the present application, the term "CIITA" generally refers to a transactivator of major histocompatibility complex Class II (MHC II). The transactivator can be a protein including an acid transcription activation domain, four LRRs (a leucine-enriched repetitive sequence) and a GTP binding domain. The protein can be located within the cell nucleus as a positive modulator of gene transcription of the major histocompatibility complex Class II (MHCII), and called as "primary control factor" for the expression of these genes. The protein can also bind to GTP and transport itself to the nucleus by binding to GTP, where it usually functions by acetyltransferase (AT) activity in a coactivator-like manner. In humans, the protein is encoded by the gene located at 16p13.13 (e.g., information as shown by HGNC:7067), which can produce several isoform transcript variants encoding different isoforms.

In the present application, the term "wild-type cell" generally refers to naturally-occurring or naturally derived cells.

In the present application, the term "T cells" generally refers to thymus-derived cells that participate in immune responses mediated by various cells.

In the present application, the term "nucleic acid" or "polynucleotide" or "nucleic acid molecule" generally refers to deoxyribonucleic acid (DNA) or ribonucleic acid (RNA) and a polymer of single stranded or double stranded formats. Unless specifically defined, the term may include nucleic acids which include naturally-occurring nucleotide analogues that have similar binding properties to a reference nucleic acid (e.g., showing the sequence information) and are metabolized in a manner similar to naturally occurring nucleotides. Unless otherwise specified, the sequence of a nucleic acid can include conservatively modified variants thereof, such as, degenerate codon substitution, alleles, direct homologs, SNPs and complementary sequences, as well as explicitly specified sequences.

In the present application, the term "expression" generally refers to the transcription and/or translation of a specific nucleotide sequence.

In the present application, the term "gene mutation" generally refers to a variation in the composition or arrangement sequence of base pairs in the structure of a gene, e.g., point mutations caused by change of a single base, or deletion, duplication, insertion of a plurality of bases, etc.

In the present application, the term "gene silencing" generally refers to blocking the expression of certain genes through regulatory mechanisms. It includes primarily two types: one is transcriptional gene silencing (TGS) caused by factors like DNA methylation, heterochromatin, location effect, etc., and the other is post-transcriptional gene silencing (PTGS), i.e., affecting the expression of gene at the post-transcriptional level by specific intervention of target RNA. Generally, when a gene is silenced, the expression of the corresponding gene is down-regulated/reduced. When a gene is knocked out, it is usually no expressed. For example, the expression of a certain gene disappears in cells if all alleles of a certain gene are knocked out. Gene silencing is usually considered as a mechanism of gene knockdown, and the general method for gene silencing can be, e.g., RNAi.

In the present application, the term "endogenous" refers to any substance derived from or generated within organisms, cells, tissues, or systems.

In the present application, the term "exogenous" refers to any substance introduced from or generated outside organisms, cells, tissues, or systems.

In the present application, the term "antisense RNA" generally refers to a single stranded RNA complementary to a transcript mRNA (messager RNA). The antisense RNA can inhibit the expression of gene by binding to mRNA. For example, the binding of antisense RNA with target mRNA results in an increased sensitivity of the double stranded RNA molecule to RNA enzyme III, and causes the degradation of the double stranded RNA molecule. For example, the antisense RNA binds to the upstream untranslated region of mRNA, so as to directly inhibit the translation of target mRNA.

In the present application, the term "siRNA" generally refers to an abbreviation of Small Interfering RNA or Short Interfering RNA. SiRNA is a class of double stranded, noncoding RNA molecules with a length of about 18-28 base pairs, which can cause the degradation of mRNA by the complementary binding with mRNA, thereby interfering with the expression of specific genes. In some embodiments, siRNA can be a product obtained by treating a long double stranded RNA or shRNA with Dicer enzyme. In some embodiments, siRNA enters a cell and forms an RNA induced silencing complex (RISC) with another protein, the sense strand is degraded, and the antisense strand binds to a complementary target sequence, thereby achieving gene silencing.

In the present application, the term "shRNA" generally refers to an abbreviation of short hairpin RNA, that is, "short hairpin RNA". ShRNA usually includes two short reverse repeat sequences spaced by a loop sequence to form a hairpin structure. Generally, it can further include 5-6 T bases as the transcription terminator of RNA polymerase III. In some embodiments, shRNA can enter cells via viral vectors or plasmids, and be transcribed under the action of polymerase II or polymerase III. The transcripts are exported from the nucleus (usually through Exportin 5), and then transported to RISC after Dicer treatment. The sense strand is degraded, and the antisense strand can be combined with a complementary target sequence, thereby achieving gene silencing.

In the present application, the term "CRISPR/Cas system" generally refers to a group of molecules including RNA-directed nuclease or other effector molecules and gRNA molecules, which can direct and realize the modification of nucleic acid at the site of targeting sequence by RNA-directed nuclease or other effector molecules, e.g., inducing degradation of the target sequence. In some embodiments, the CRISPR system includes gRNA and a Cas protein, e.g., a Cas9 protein. The system including Cas9 or a functional variant thereof is called "Cas9 system" or "CRISPR/Cas9 system" in the present application. In some embodiments, the gRNA molecule can be complexed with the Cas molecule to form a ribonucleoprotein (RNP) complex.

In the present application, the terms "gRNA molecule" or "guide RNA", "instruction RNA", "direct RNA", "guide RNA molecule", "gRNA" can be used interchangeably, and generally refer to a nucleic acid molecule that can promote directing the RNA-directed nuclease or other effector molecules (generally combined with gRNA molecules) specifically to the target sequence. In some embodiments, the directing is achieved by the hybridization of a portion of gRNA with DNA (e.g., via a gRNA guide domain) and the binding of a portion of gRNA molecule with an RNA directed nuclease or other effector molecules (e.g., at least through gRNAtracr). In some embodiments, the gRNA molecule consists of a single continuous polynucleotide molecule, which is herein referred to as a "single guide RNA" or "sgRNA" or the like. In other embodiments, the gRNA molecule consists of a plurality of (e.g., two) polynucleotide molecules that can be associated with themselves (generally by hybridization), which is herein referred to as "double guide RNA" or "dgRNA", etc.

In the present application, the term "Cas protein" generally refers to an enzyme responsible for cutting DNA in the CRISPR/Cas system. It can include enzymes from CRISPR/Cas system Types I, II, and III, e.g., Cas3, Cas9, Cas10.

In the present application, the term "Cas9 protein" generally refers to an enzyme from the bacterial type II CRISPR/Cas system and responsible for cutting DNA. Cas9 can include the wild-type protein and functional variants thereof.

In the present application, "allele" generally refers to various forms of variations in gene sequences on loci. Locus, also known as gene site or site, refers to a fixed position on a chromosome, such as the location of a gene. The arrangement of loci in a genome is called genetic map.

In the present application, the term "chimeric antigen receptor (CAR)" generally refers to an antigen receptor fused by fusing an antigen binding region of an antibody which recognizes a tumor associated antigen (TAA) or a binding fragment of other target molecules with an "immune receptor tyrosine-based activation motifs (ITAM, typically CD3ζ or FcεRIγ) of an intracellular signal domain. For example, the basic structure of CAR can include an antigen binding domain of a tumor-associated antigen (TAA) or other target molecules (typically, an scFv originated from the antigen binding region of a monoclonal antibody), an extracellular hinge region, a transmembrane region, and an immunoreceptor tyrosine-based activation motif (ITAM) of an intracellular immune receptor.

In the present application, the term "binding domain" generally refers to a domain that (specifically) binds to a given target epitope or a given target site of a target molecule (e.g., an antigen), interacts with the given target epitope or the given target site, or recognizes the given target epitope or the given target site.

In the present application, the term "specific binding" generally refers to a measurable and reproducible interaction, such as, the binding between a target and an antibody, which can determine the presence of a target in the presence of heterogeneous populations of molecules (including biomolecules). For example, antibodies that specifically bind to targets (which can be epitopes) are antibodies that bind the target(s) with greater compatibility, affinity, easiness, and/or duration than other targets. In some embodiments, the antibody specifically binds to an epitope on a protein that is conserved in proteins of different species. In another embodiment, the specific binding includes but is not limited to exclusive binding.

In the present application, the term "transmembrane domain" generally refers to a polypeptide or protein which is encoded at a DNA level by an exon including at least an extracellular region, a transmembrane region, and an intracellular region. The transmembrane domain generally includes three different structural regions: N-terminal extracellular region, middle conserved transmembrane extension region, and C-terminal cytoplasmic region. The transmembrane domain may further include an intracellular region or a cytoplasmic region.

In the present application, the term "hinge region" generally refers to a region located between the binding domain and the transmembrane domain in the CAR structure. The hinge region usually comes from IgG family, such as IgG1 and IgG4, and some from IgD and CD8. Generally, the hinge region has a certain degree of flexibility, which affects the spatial constraints between the CAR molecule and its specific target, thereby affecting the contact between CAR T cells and tumor cells.

In the present application, the term "costimulatory" generally refers to a source of the second signal of lymphocyte activation, which is usually generated by an interaction of costimulatory molecules on the surface of immune cells (between T cells/B cells or between antigen presenting cells/T cells) involved in adaptive immunity with their receptors. For example, the complete activation of T cells depends on dual signaling and the action of cytokine. The first signal of T cell activation is derived from the specific binding of its receptors with the antigens, that is, the recognition of T cells to the antigens; and the second signal of T cell activation is derived from the costimulatory molecule, that is, the interaction of the costimulatory molecules of the antigen presenting cells with the corresponding receptors on the surfaces of T cells.

In the present application, the term "costimulatory domain" generally refers to an intracellular portion of the corresponding receptor of the costimulatory molecule, which can transduce a costimulatory signal (also known as the second signal). For example, in CAR-T cells, the costimulatory domain derived from CD137 (or receptors of other costimulatory molecules) can be activated after the binding of the extracellular binding domain in the CAR structure with the corresponding antigen, thereby transducing a costimulatory signal.

In the present application, the term "primary signal transduction domain" generally refers to an amino acid sequence within a cell that can generate signals which promote the immune effector function of CAR-containing cells such as CAR-T cells. Examples of the immune effector functions in, e.g., CAR-T cells can include cell lysis activity and auxiliary activity, including cytokine secretion. In some embodiments, the primary signal transduction domain transduces the effector functional signals and directs the cells to perform the specialization function. Although the primary signal transduction domain can be used in its entirety, it is not necessary to use the entire chain in many cases. As for the use of a truncated portion of the primary signal transduction domain, such truncated portion can be used to replace the intact chain, as long as it can transduce the effector functional signals. The term "primary signal transduction domain" is thus intended to encompass any truncated portion of an intracellular signal transduction domain that is sufficient to transduce the effector functional signals.

In the present application, the term "tumor antigen" generally refers to a molecule that is expressed on the surface of a tumor cell in its entirety or as a fragment and can be used to preferentially direct a drug to the tumor cell (e.g., proteins, saccharides, or lipids). In some embodiments, the tumor antigen can be a marker expressed by both normal cells and cancer cells, e.g., genealogical markers, such as, CD19 on B cells. In some embodiments, the tumor antigen can be cell surface molecules with over-expression in tumor cells, e.g., 1-fold, 2-fold, 3-fold, or more over-expression, as compared with that in normal cells. In some embodiments, the cell surface molecules are abnormally expressed in tumor cells, e.g., including deletion, addition, or mutation of molecules as compared with the corresponding molecules expressed in normal cells. In some embodiments, the tumor antigen can be exclusively expressed on the surface of tumor cells in its entirety or as a fragment (e.g., MHC/peptide), and not synthesized or expressed on the surface of normal cells. In some embodiments, the CARs in the present application can include an antigen binding domain binding to the MHC-presenting peptide, such as, an antibody or a fragment of antibody. In general, endogenous protein-derived peptides bind to major histocompatibility complex (MHC) Class I molecules, and are recognized by the T cell receptors (TCR) on CD8+T lymphocytes. MHC Class I complexes are constitutively expressed by all the karyocytes. Virus specific and/or tumor specific peptide/MHC complexes can be used as a unique class of cell surface targets for immunotherapy. (see, e.g., Sastry et al., J Virol.2011 85(5):1935-1942; Sergeeva et al., Blood, 2011 117(16):4262-4272; Verma et al., J Immunol 2010 184(4):2156-2165; Willemsen et al., Gene Ther 2001 8(21):1601-1608; Dao et al., Sci Transl Med 2013 5(176):176ra33; Tassev et al., Cancer Gene Ther 2012 19(2):84-100).

In the present application, the term "monoclonal antibody" generally refers to antibodies obtained from a population of substantially homogeneous antibodies. Namely, all antibodies constituting the population are the same, except for potential natural mutation and/or post-translational modification (such as isomerization and amidation) that may be present in a very small amount. The monoclonal antibody is highly specific and targets a single antigenic site. Unlike typical polyclonal antibody preparations containing different antibodies targeting different determinants (epitopes), each type of monoclonal antibody targets a single determinant on the antigen. In addition to their specificity, the advantages of monoclonal antibodies rely on that they are synthesized from hybridoma cultures and are not contaminated by other immunoglobulins. The modifier "monoclonal" indicates that the antibody obtains characteristics from a population of substantially homogeneous antibodies, and should not be interpreted as requiring generating an antibody by any specific method.

In the present application, the term "polyclonal antibody" generally refers to a composition of various antibody molecules. The polyclonal antibodies can bind to or be reacted with a plurality of different specific antigen determinants on a single antigen or different antigens. The variation degree of antigenic specificity of the polyclonal antibodies is located within the variable regions of a single antibody constituting the polyclonal antibodies, such as, in the complementary determining region (CDR) 1, CDR2, and CDR3 regions. For example, the polyclonal antibodies can be prepared by immunizing animals with target sFGFR or a part thereof. For example, the polyclonal antibodies can be prepared by mixing multiple monoclonal antibodies with the desired target sFGFR specificity.

In the present application, the term "human antibody" generally refers to an antibody with variable and constant regions derived from the sequence of immunoglobulin of human germ line. Human antibodies are well known in the prior art (e.g., please refer to van Dijk, M.A. and van de Winkel, J.G., Curr.Opin.Chem.Biol.5(2001)368-374). The human antibodies can also be generated in transgenic animals (e.g., mice) which can generate a complete or selected set of human antibodies in absence of generated endogenous immunoglobulin after immunization (e.g., Jakobovits, A. et al., Proc.Natl.Acad.Sci.USA 90(1993)2551-2555; Jakobovits, A. et al., Nature 362(1993)255-258; Brueggemann, M. et al., YearImmunol.7(1993)33-40). The human antibodies can also be generated in a phage display library (e.g., Hoogenboom, H.R. and Winter, G., J.Mol.Biol.227(1992)381-388; Marks, J.D. et al., J.Mol.Biol.222(1991)581-597). The term "human antibody" can also include antibodies modified in the constant regions.

In the present application, the term "humanized antibody" generally refers to an antibody which includes sequences of heavy chain variable regions and light chain variable regions derived from non-human species (e.g., mice), but in which at least a portion of the VH and/or VL sequences have been changed to be similar to the human germline variable sequences. For example, the term "humanized antibody" is an antibody or a variant, derivative, analogue or fragment thereof that can bind to a related antigen with immune specificity and includes a framework region (FR) which includes substantially an amino acid sequence of a human antibody and a complementary determining region (CDR) which includes substantially an amino acid sequence of a non-human antibody. In the context of CDR, the term "substantially" means that the amino acid sequence of CDR has at least 80%, e.g., at least 85%, at least 90%, at least 95%, at least 98% or at least 99% identity with an amino acid sequence of CDR of a non-human antibody. The humanized antibodies include substantially at least one, typically two variable domains (Fab, Fab', F(ab')2, FabC, Fv), wherein all or substantially all CDR regions correspond to the CDR region of a non-human immunoglobulin and all or substantially all framework regions are frame regions with consensus sequence of human immunoglobulin. In some embodiments, the humanized antibody can further include at least a portion of a constant region of immunoglobulin (Fc), typically a constant region of human immunoglobulin.

In the present application, the term "single domain antibody" generally refers to a class of antibodies in which a light chain of antibody is deleted and a heavy chain variable region is contained. It is also known as nanobody due to its low molecular weight. Single domain antibodies were initially found in camel family animals, and then similar antibodies were found in nurse shark, big star shark, skate and other cartilaginous fishes. For example, antibody in which the light chain and the CH1 region of the heavy chain constant region of a conventional antibody are deleted is called heavy chain antibody (HcAb), which are ubiquitous in all kinds of camel family. For example, a heavy chain antibody called as Ig new antigen receptor is abbreviated as IgNAR, which is composed of two identical heavy chains including five constant regions and one variable region. The variable regions of the heavy chain antibody are merely composed of the variable region of the heavy chain of the antibody. Like the Fab of a conventional antibody, the variable region can specifically bind to an antigen, so that the heavy antibody can play the same role as a conventional antibody.

In the present application, the term "tumor" generally refers to a neoplasm or solid lesion formed by abnormal cell growth. In the present application, the tumor can be a solid tumor or a non-solid tumor. In some embodiments, a visible lump that can be detected by clinical examinations such as, X-ray radiography, CT scanning, B-ultrasound or palpation can be called solid tumor, while a tumor that cannot be seen or touched by X-ray, CT scanning, B-ultrasound and palpation, such as leukemia, can be called non-solid tumor.

In the present application, the term "CD" (i.e., Cluster of differentiation), also called differentiation population, generally refers to cell surface molecules used to recognize immune antibody labels. CD molecules have many applications, typically as important receptors or ligands of cells. Some CDs can participate in the signal cascade of cells, thereby changing the behavior of cells. Some CDs are independent of cell signal transduction, but have other functions, such as cell adhesion. Until April 21, 2016, the total number of human CD molecules is 371, such as, CD28, CD3e, CD45, CD4, CD5, CD8, CD9, CD16, CD22, CD33, CD37, CD64, CD80, CD86, CD134, CD137 and CD154 used as the source of transmembrane domain, and CD137, CD28, CD134 (OX-40) and CD278 (ICOS) as the source of costimulatory domain in the present application.

In the present application, the term "pharmaceutically acceptable carrier" generally refers to a pharmaceutically acceptable substance, composition, or vehicle involved in carrying, storing, transferring, or administering a cell preparation, e.g., liquids, semi-solid or solid fillers, diluents, osmotic agents, solvent, or encapsulating substances. The pharmaceutically acceptable carrier can include a pharmaceutically acceptable salt, wherein the term "pharmaceutically acceptable salt" includes salts of active compounds prepared by using a relatively nontoxic acid or base, e.g., sodium chloride, depending on the cell nature of the present application. The pharmaceutically acceptable carrier can further include organic acids (e.g., lactic acid), bioactive substances (e.g., polypeptides, antibodies, and the like) and antibiotics (e.g., penicillin, streptomycin), etc. The pharmaceutically acceptable carrier can further include a hydrogel, such as, a hydrogel containing polyacrylamide. The pharmaceutically acceptable carrier can include storage solution, cryopreservation solution, injection, etc., which can be used for cells. In general, the pharmaceutically acceptable carrier can maintain the activity of the cells carried by the carrier without hindering its therapeutic efficacy. The pharmaceutically acceptable carrier can also contribute to the storage, transportation, proliferation and migration of cells, and is suitable for clinical application.

In the present application, the term "composition" generally refers to a composition suitable for administration to patients, human patients. For example, the composition of the present application can include the immunologic effector cells of the present application, and optionally a pharmaceutical acceptable carrier. In some embodiments, the acceptable ingredients of the composition are nontoxic to the recipients at the dose and concentration as used. The composition of the present application includes, but is not limited to, liquid, frozen, and lyophilized compositions.

In the present application, the term "allogeneic therapy" generally refers to a therapy of administering organs, tissues, cells, etc. which do not come from the subject or patient to achieve a therapeutic purpose.

In the present application, the term "include/including" generally refers to encompassing clearly specified features, but does not exclude other elements.

In the present application, the term "about" generally refers to a variation within 0.5% to 10% of a given value, e.g., a variation within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of a given value.

In an aspect, the present application relates to a modified immunologic effector cell, wherein the expression and/or activity of the TRAC gene and the HLA-A gene are/is down-regulated, the expression and/or activity of the B2M gene are/is not down-regulated, and the expression and/or activity of the CIITA gene are/is not down-regulated, as compared with the expression and/or activity of the corresponding genes in corresponding cells which are not modified.

In another aspect, the present application further provides a method of preparing the modified immunologic effector cell of the present application, including the following steps: as compared with the expression and/or activity of the corresponding genes in corresponding cells which are not modified, down-regulating the expression and/or activity of the TRAC gene and the HLA-A gene in the immunologic effector cell, not down-regulating the expression and/or activity of the B2M gene, and not down-regulating the expression and/or activity of the CIITA gene.

In the present application, the modification results in down-regulation of expression and/or activity of two genes, wherein the two genes consist of the TRAC gene and the HLA-A gene.

### Immune Effector Cells

In the present application, the immune effector cells can include plasmacytes, T cells, B cells, natural killer (NK) cells, natural killer T (NKT) cells, mastocytes, and/or bone marrow-derived phagocytes.

In the present application, the plasmacytes refer to effector B cells/antibody secreting cells, which can include proto-plasmacytes, proplasmacytes, Russell bodies, Dutcher bodies, flame-like cells, etc.

In the present application, the B cells refer to all types of B cells except plasmacytes, such as, pre-B cells, immature B cells, mature B cells, activated B cells, etc.

In the present application, the T cells can include: helper T cells (Th) that can assist in humoral immune and cellular immunity; suppressor T cells (Ts) that can suppress cellular immunity and humoral immunity; effector T cells (Te) that can release lymphokines; cytotoxic T cells (Tc) that can kill targets; delayed type hypersensitivity T cells (Td) that can participate in the action of Type IV allergy; amplifying T cells (Ta) that can act on Th and Ts to expand the immune effect; virgin or natural T cells that can differentiate into effector T cells or memory T cells upon exposure to an antigen; and memory T cells (Tm) that can remember specific antigen stimulations.

For example, the cytotoxic T cells can include a cell surface marker CD8+.

For example, the helper T cells can include a cell surface marker CD4+.

In the present application, the corresponding cells which are not modified can include wild-type cells and/or engineered cells. The wild-type cells can include naturally occurring cells or naturally derived cells, such as, plasmacytes, T cells, B cells, natural killer (NK) cells, natural killer T (NKT) cells, mastocytes and/or bone marrow-derived phagocytes which are isolated from human body, or immune effector cells which are differentiated by inducing precursor cells or pluripotent cells isolated from human body. The engineered cells can be plasmacytes, T cells, B cells, natural killer (NK) cells, natural killer T (NKT) cells, mastocytes and/or bone marrow-derived phagocytes which are isolated from human body or differentiated by inducing precursor cells or pluripotent cells isolated from human, and further engineered. For example, the human body is a healthy human body. For example, the healthy human body can include those without tumors or immune system-associated diseases or disorders.

In the present application, the artificial engineering can be those which do not target TRAC gene, HLA-A gene, B2M gene and the CIITA gene. For example, the artificial engineering does not affect the expression and/or activity of the TRAC gene, HLA-A gene, B2M gene and the CIITA gene. Said not affecting the expression and/or activity of TRAC gene, HLA-A gene, B2M gene and CIITA gene means that as compared with the corresponding cells which are isolated from human body, or corresponding cells which are differentiated by inducing precursor cells or pluripotent cells isolated from human body and are not further engineered, the expression and/or activity of the TRAC gene, HLA-A gene, B2M gene and the CIITA gene are not changed and not completely consistent. For instance, as measured by conventional technical means in the art, there is no significant difference (P > 0.05) in the mRNA quantitative analysis of TRAC gene, HLA-A gene, B2M gene and the CIITA gene between the compared cells. By way of example, as measured by the conventional technical means in the art, there is no significant difference (P > 0.05) in the corresponding polypeptide/protein quantitative analysis of TRAC gene, HLA-A gene, B2M gene and the CIITA gene between the compared cells. For example, the artificial engineered cells can include CAR-T cells. For example, the CAR-T cells can include those in which the CAR molecule includes a binding domain targeting a molecule selected from: CD19, PSCA, CD123, CD20, CEA, FAP, CD133, EGFR, EGFRVIII, BCMA, PSMA, Her2, CA125, EphA2, C-met, L1CAM, VEGFR, CS1, ROR1, EC, NY-ESO-1, MUC1, LewisY, GPC3, GD2, DLL3, CD99, 5T4, CD22, CD30, CD33, CD138 and/or CD171, CD19, CD133, CD123, CD22, CD30, CD171, CA125, C-met, L1CAM, EC, DLL3, CD99, CS1, 5T4, CD138, CS-1 (also known as CD2 subclass 1, CRACC, SLAMF7, CD319 or 19A24), C-type lectin-like molecule-1 (CLL-1 or CLECL1), CD33, epidermal growth factor receptor variant III (EGFRvIII), ganglioside G2 (GD2), ganglioside GD3, TNF receptor family member B cell mature antigen (BCMA), Tn antigen (e.g., Tn Ag, GalNAcα-Ser/Thr), prostate-specific membrane antigen (PSMA); receptor tyrosine kinase-like orphan receptor 1 (ROR1), Fms-like tyrosine kinase 3 (FLT3); tumor-associated glycoprotein 72 (TAG72), CD38, CD44v6, carcino-embryonic antigen (CEA), epithelial cell adhesion molecule (EPCAM), B7H3 (CD276), KIT (CD117), interleukin-13 receptor subunit α-2 (IL-13Ra2 or CD213A2), mesothelin, interleukin 11 receptor α (IL-11Ra), prostate stem cell antigen (PSCA), protease serine 21, vascular endothelial growth factor receptor 2 (VEGFR2), Lewis (Y) antigen, CD24, platelet-derived growth factor receptor β (PDGFR-β), stage-specific embryonic antigen-4 (SSEA-4), CD20, folate receptor α, receptor tyrosine-protein kinase ERBB2 (Her2/neu), cell surface-associated mucoprotein 1 (MUC1), epidermal growth factor receptor (EGFR), neurocyte adhesion molecule (NCAM), prostase, prostate acid phosphatase (PAP), elongation factor 2 mutant (ELF2M), ephrin B2, fibroblast activated protein α (FAP), insulin-like growth factor 1 receptor (IGF-I receptor), carbonic anhydrase IX (CAIX), proteasome (e.g., proteasome, giantin factor) subunit B-type 9 (LMP2), glycoprotein 100 (gp100), oncogene fusion protein (bcr-abl) composed of breakpoint cluster region (BCR) and Abelson murine leukemia virus oncogene homolog 1 (Abl), tyrosinase, ephrin A-type receptor 2 (EphA2), fucosyl GM1; sialyl Lewis adhesion molecule (sLe), transglutaminase 5 (TGS5), high molecular weight-melanoma-associated antigen (HMWMAA), o-acetyl-GD2 ganglioside (OAcGD2), folate receptor β, tumor endothelial marker 1 (TEM1/CD248), tumor endothelial marker 7-associated (TEM7R), tight junction protein 6 (CLDN6), thyrotropin receptor (TSHR), G-protein-coupled receptor C-class Group 5 Member D (GPRC5D), chromosome X ORF 61 (CXORF61), CD97, CD179a, anaplastic lymphoma kinase (ALK), polysialic acid, placental specificity 1 (PLAC1), hexose moiety of globoH glycoceramide (GloboH), mammary gland differentiation antigen (NY-BR-1), uroplakin 2 (UPK2), hepatitis virus A cell receptor 1 (HAVCR1), adrenaline receptor β3 (ADRB3), pannexin 3 (PANX3), G-protein-coupled receptor 20 (GPR20), lymphocyte antigen 6 complex loci K9 (LY6K), olfactory receptor 51E2 (OR51E2), TCRγ variable ORF protein (TARP), Wilm tumor protein (WT1); cancer/testis antigen 1 (NY-ESO-1), cancer/testis antigen 2 (LAGE-1a), melanoma-associated antigen 1 (MAGE-A1), ETS translocation variant gene 6 located on chromosome 12p (ETV6-AML), sperm protein 17 (SPA17), X antigen family member 1A (XAGE1), angiogenin cell surface receptor 2 (Tie 2), melanoma cancer/testis antigen-1 (MAD-CT-1), melanoma cancer/testis antigen-2 (MAD-CT-2), Fos-associated antigen 1, p53, p53 mutant, prostate-specific protein (prostein), prostate cancer tumor antigen-1 (PCTA-1 or galectin 8), T cell-recognized melanoma antigen 1 (MelanA or MARTI); rat sarcoma (Ras) mutant, human telomerase reverse transcriptase (hTERT), sarcoma translocation breakpoint, melanoma anti-apoptosis protein (ML-IAP), ERG (transmembrane protease, serine 2 (TMPRSS2) ETS fusion gene), N-acetylglucosaminyltransferase V (NA17), paired frame protein Pax-3 (PAX3), androgen receptor, cyclin B1, v-myc avian myelocytosis virus oncogene neuroblastoma-derived homologs (MYCN), Ras homolog family member C (RhoC), tyrosinase-associated protein 2 (TRP-2), cytochrome P450 1B 1 (CYP1B1), T cell-recognized squamous cell cancer antigen 3 (SART3), paired frame protein Pax-5 (PAX5), proacrosomal protein binding protein sp32 (OY-TES1), lymphocyte-specific protein tyrosine kinase (LCK), A kinase anchorin 4 (AKAP-4), synovial sarcoma X breakpoint 2 (SSX2), receptor of advanced glycation end products (RAGE-1), legumain, human papilloma virus E6 (HPV E6), human papilloma virus E7 (HPV E7), intestinal carboxylesterase, mutated heat shock protein 70-2 (mut hsp70-2), CD79a, CD79b, CD72, leukocyte-associated immunoglobulin-like receptor 1 (LAIR1), Fc fragment of IgA receptor (FCAR or CD890), leukocyte immunoglobin-like receptor superfamily A member 2 (LILRA2), CD300 molecule-like family member f (CD300LF), C-type lectin domain family 12 member A (CLEC12A), bone marrow stromal cell antigen 2 (BST2), EGF-like modulus-containing mucoprotein-like hormone receptor-like 2 (EMR2), lymphocyte antigen 75 (LY75), glypican-3 (GPC3), Fc receptor-like 5 (FCRL5) and/or immunoglobulin λ-like peptide 1 (IGLL1).

### TRAC Gene, HLA-A Gene, B2M Gene and the CIITA Gene

In the present application, the expression and/or activity of the TRAC gene and the HLA-A gene are/is down-regulated, the expression and/or activity of the B2M gene are/is not down-regulated, and the expression and/or activity of the CIITA gene are/is not down-regulated, as compared with corresponding wild type cells.

In the present application, as compared with a wild type cell, the expression and/or activity of the two genes are/is down-regulated, wherein the two genes consist of the TRAC gene and the HLA-A gene.

In the present application, the down-regulation of the expression level and/or activity of the genes includes a down-regulation of expression and/or activity of a nucleic acid molecule encoding the genes; and/or a down-regulation of expression and/or activity of a protein product encoded by the genes.

For example, the protein product can include a polypeptide.

In the present application, the down-regulated expression and/or activity of the TRAC gene and the HLA-A gene or the down-regulating the expression and/or activity of the TRAC gene and the HLA-A gene in the immune effector cells can include that, as compared with the corresponding cells which are not modified, the modification changes the structure of the nucleotide sequence of the TRAC gene and the HLA-A gene. The nucleotide sequence can include coding or non-coding region, such as, cis-regulatory element sequence, exon sequence, wherein the cis-regulatory element can include a promotor. The change can include deletion of partial or full sequences, insertion of exogenous fragments, mutation of base site, or the like. The inserted exogenous fragments can substitute or disrupt the sequence structures of the TRAC gene and the HLA-A gene, so that they cannot be normally translated. The mutation of base sites can include frameshift mutations, missense mutations, nonsense mutations, and the like. The change can include the modification of nucleotide sequence with a chemical group, such as, methylation, etc. The change of the structure of the nucleotide sequence can be detected by gene sequencing, such as, Sanger sequencing, bisulfite sequences, and the like.

In the present application, the down-regulated expression and/or activity of the TRAC gene and the HLA-A gene or the down-regulating the expression and/or activity of the TRAC gene and the HLA-A gene in the immune effector cells can further include that, as compared with the corresponding cells which are not modified, the modification enables the mRNA level of the TRAC gene and the HLA-A gene to be decreased. The mRNA level can be determined by experimental methods and biomedical methods that are well known by persons skilled in the art, e.g., in situ hybridization of molecular probe, real-time fluorescent quantitative PCR (qPCR, RT-PCR). The real-time fluorescent quantitative PCR can include SYBR green method, TaqMan method, two hybrid probe method, and molecular beacon method. The detecting results of the mRNA level allow unavoidable errors in experiments or statistics, which can be well known in the art. The error can be in a range of ±10%, e.g., in a range of ±8%, ±6%, ±5%, ±4%, or ±3%. The mRNA level is decreased by at least 30%, e.g., 35%, 40%, 45%, 50%, 55%, 60%, 65%, 70%, 75%, 80%, 85%, 90%, 95%, and 99%. For example, the mRNA of the TRAC gene and the HLA-A gene is undetectable in the modified immune effector cells.

In the present application, the down-regulated expression and/or activity of the TRAC gene and the HLA-A gene or the down-regulating the expression and/or activity of the TRAC gene and the HLA-A gene in the immune effector cells can further include that, as compared with the corresponding cells which are not modified, the modification enables the polypeptide level expressed by the TRAC gene and the HLA-A gene to be decreased. The polypeptide refers to polypeptides having the same structure and function as those generated by the expression of the TRAC gene and the HLA-A gene in the corresponding cells which are not modified, while polypeptides having changed structure and function generated by the expression of the TRAC gene and the HLA-A gene due to the change of nucleotide sequence are not used for comparison. The polypeptide level can be determined by experimental methods and biometric methods that are well known by persons skilled in the art, such as, flow cytometry, enzyme-linked immunosorbent assay (ELISA), cell immunofluorescence staining, and Western blotting (WB). The detecting results of the polypeptide level allow unavoidable errors in experiments or statistics, which can be well known in the art. The error can be in a range of ±10%, e.g., in a range of ±8%, ±6%, ±5%, ±4%, or ±3%. The polypeptide level is decreased by at least 30%, e.g., 35%, 40%, 45%, 50%, 55%, 60%, 65%, 70%, 75%, 80%, 85%, 90%, 95%, and 99%. For example, the polypeptide expressed by the TRAC gene and the HLA-A gene is not detectable in the modified immune effector cells.

In the present application, the down-regulated expression and/or activity of the TRAC gene and the HLA-A gene or the down-regulating the expression and/or activity of the TRAC gene and the HLA-A gene in the immune effector cells can refer to knockdown or knockout of the expression of the TRAC gene and the HLA-A gene or implementation of other operations that disrupt the function of TRAC protein and the HLA-A protein.

In the present application, the expression and/or activity of the B2M gene being not down-regulated and the expression and/or activity of the CIITA gene being not down-regulated, or not down-regulating the expression and/or activity of the B2M gene, and not down-regulating the expression and/or activity of the CIITA gene can include, as compared with the corresponding cells which are not modified, the mRNA level of the B2M gene and the CIITA gene are not decreased. The mRNA level can be determined by experimental methods and biometrical methods that are well known by persons skilled in the art, e.g., in situ hybridization of molecular probe, real-time fluorescent quantitative PCR (qPCR, RT-PCR). The real-time fluorescent quantitative PCR can include SYBR green method, TaqMan method, two hybrid probe method, and molecular beacon method. The detecting results of the mRNA level allow unavoidable errors in experiments or statistics, which can be well known in the art. The error can be in a range of ±10%, e.g., in a range of ±8%, ±6%, ±5%, ±4%, or ±3%. The mRNA level being not decreased can include a case that the level is increased or unchanged. The level being unchanged can include a case that there is no significant difference in the quantitative analysis of the corresponding mRNA of B2M gene and CIITA gene between the two (P > 0.05).

In the present application, the expression and/or activity of the B2M gene being not down-regulated, and the expression and/or activity of the CIITA gene being not down-regulated, or not down-regulating the expression and/or activity of the B2M gene and not down-regulating the expression and/or activity of the CIITA gene can include that, as compared with the corresponding cells which are not modified, the level of the polypeptide expressed by the B2M gene and the CIITA gene is not decreased. The polypeptide refers to polypeptides having the same structure and function as those generated by the expression of the B2M gene and the CIITA gene in the corresponding cells which are not modified. The polypeptide level can be determined by experimental methods and biometric methods that are well known by persons skilled in the art, such as, flow cytometry, enzyme-linked immunosorbent assay (ELISA), cell immunofluorescence staining, and Western blotting (WB). The detecting results of the polypeptide level allow unavoidable errors in experiments or statistics, which can be well known in the art. The error can be in a range of ±10%, e.g., in a range of ±8%, ±6%, ±5%, ±4%, or ±3%. The polypeptide level being not decreased can include a case that the level is increased or unchanged. The level being unchanged can include a case that there is no significant difference in the quantitative analysis of the corresponding polypeptide of B2M gene and CIITA gene between the two (P > 0.05).

In the present application, the expression and/or activity of the B2M gene being not down-regulated, and the expression and/or activity of the CIITA gene being not down-regulated, or not down-regulating the expression and/or activity of the B2M gene and not down-regulating the expression and/or activity of the CIITA gene can include a case that a manual intervention is not performed on the corresponding cells which are not modified against the CIITA gene and B2M gene and mRNA and/or polypeptide encoded by the CIITA gene and B2M gene. For example, the manual intervention can include introducing a nucleotide molecule or other compounds which can target the CIITA gene and the B2M gene or an mRNA molecule encoded thereby and which can change the structure or level thereof into the corresponding cells which are not modified.

In the present application, the expression and/or activity of the B2M gene being not down-regulated and the expression and/or activity of the CIITA gene being not down-regulated, or not down-regulating the expression and/or activity of the B2M gene and not down-regulating the expression and/or activity of the CIITA gene can include that, as compared with the corresponding cells which are not modified, the structure of the nucleotide sequence of the B2M gene and the CIITA gene are not changed. The structure of the nucleotide sequence being unchanged can be determined by gene sequencing, such as, Sanger sequencing, sulfite sequencing, etc. The nucleotide sequence being unchanged can include not only a case that there is no manual change, but also a case that there is naturally occurring change which would not affect the function.

In the present application, the expression and/or activity of the B2M gene being not down-regulated, and the expression and/or activity of the CIITA gene being not down-regulated, or not down-regulating the expression and/or activity of the B2M gene and not down-regulating the expression and/or activity of the CIITA gene can refer to a case that there is no modification with B2M gene and proteins encoded thereby, CIITA gene or proteins encoded thereby as targets.

In the present application, the expression level and/or activity of at most 2 alleles of the HLA-A gene are down-regulated. For example, the 2 alleles can be a pair of alleles of HLA-A gene in the immune effector cells. For example, the expression level and/or activity of 1 allele in the HLA-A gene is down-regulated.

In the present application, the TRAC gene can include a gene as shown by HGNC: 12029 and all allele types thereof. For example, the TRAC gene and all allele types thereof can include the TRAC genotypes capable of existing in the immune effector cells. For example, the TRAC gene can include a nucleotide sequence as shown in SEQ ID No. 55.

For example, the TRAC gene can include a nucleotide sequence that is derived from human being and has 80% or greater, e.g., 85% or greater, 90% or greater, 95% or greater, 96% or greater, 97% or greater, 98% or greater, 99% or greater of identity as the nucleotide sequence as shown in SEQ ID No. 55.

In the present application, the HLA-A gene can include a gene as shown by HGNC:4931 and all allele types thereof. For example, the HLA-A gene can include all the HLA-A allele types included in the IMGT/HLA Database Version 3.38.0 (https://www.ebi.ac.uk/ipd/imgt/hla/) and named by the WHO HLA Factor Nomenclature Committee. The HLA-A allele genotypes and the sequence information thereof disclosed in the IMGT/HLA data 3.38.0 are incorporated herein by reference.

For example, the HLA-A allele can include any one or more of A^{∗}02, A^{∗}24, A^{∗}01, A^{∗}03, A^{∗}32, A^{∗}11, A^{∗}26, A^{∗}68, A^{∗}23, A^{∗}29, A^{∗}31, A^{∗}33, A^{∗}25, A^{∗}43, A^{∗}74, A^{∗}30, A^{∗}69.

For example, the HLA-A allele can include any one or more of A^{∗}02:01, A^{∗}03:01, A^{∗}01:01, A^{∗}24:02, A^{∗}68:01, A^{∗}11:01, A^{∗}31:01:02, A^{∗}29:02, A^{∗}32:01, A^{∗}26, A^{∗}23:01, A^{∗}30:02, A^{∗}25:01, A^{∗}33:03.

For example, the HLA-A allele can include any one or more of A^{∗}02:01:01, A^{∗}01:01:01, A^{∗}03:01:01, A^{∗}24:02:01, A^{∗}11:01:01, A^{∗}32:01:01, A^{∗}29:02:01, A^{∗}31:01:02, A^{∗}23:01:01, A^{∗}26:01:01, A^{∗}68:01:02, A^{∗}30:01:01, A^{∗}68:02:01, A^{∗}25:01:01, A^{∗}68:01:01,
and A^{∗}02:05:01.

For example, the HLA-A allele can include any one or more of A^{∗}02, A^{∗}30, A^{∗}03, A^{∗}01, A^{∗}24, A^{∗}32, A^{∗}68, A^{∗}11, A^{∗}26, A^{∗}23, A^{∗}31, A^{∗}25.

For example, the HLA-A allele can include any one or more of A^{∗}02:01, A^{∗}03:01, A^{∗}24:02, A^{∗}01:01, A^{∗}11:01, A^{∗}26:01, A^{∗}25:01, A^{∗}68:01, A^{∗}32:01, A^{∗}31:01.

For example, the HLA-A allele can include any one or more of A^{∗}24, A^{∗}33, A^{∗}02, A^{∗}11, A^{∗}26, A^{∗}31, A^{∗}01, A^{∗}24:02, A^{∗}02:01, A^{∗}33:03, A^{∗}11:01, A^{∗}26:01, A^{∗}02:06, A^{∗}31:01:02, A^{∗}26:03, A^{∗}26:02, A^{∗}02:07, A^{∗}01:01, A^{∗}02:10, A^{∗}03:01.

For example, the HLA-A allele can include A^{∗}02, A^{∗}24, A^{∗}33, A^{∗}11, A^{∗}26, A^{∗}31, A^{∗}30, A^{∗}03, A^{∗}01, A^{∗}32, A^{∗}29, A^{∗}68, A^{∗}23, A^{∗}25, A^{∗}34, A^{∗}36, A^{∗}43, A^{∗}66, A^{∗}74.

For example, the HLA-A allele can include any one or more of A^{∗}24:02, A^{∗}33:03, A^{∗}02:01, A^{∗}11:01, A^{∗}02:01, A^{∗}31:01, A^{∗}26:01, A^{∗}02:07, A^{∗}30:01, A^{∗}26:02, A^{∗}01:01,

A^{∗}03:01, A^{∗}30:04, A^{∗}26:03.

For example, the HLA-A allele can include any one or more of A^{∗}02:01, A^{∗}11:01, A^{∗}24:02, A^{∗}30:01, A^{∗}26:01, A^{∗}23:01, A^{∗}02:07, A^{∗}02:06, A^{∗}03:01, A^{∗}01:01, A^{∗}31:01:02, A^{∗}33:03, A^{∗}32:01, A^{∗}68:01, A^{∗}02:03, A^{∗}02:05.

For example, the HLA-A allele can include any one or more of A^{∗}03:01, A^{∗}02:01, A^{∗}23:01, A^{∗}01:01, A^{∗}30:02, A^{∗}30:01, A^{∗}33:03, A^{∗}29:02, A^{∗}74:01, A^{∗}36:01, A^{∗}24:02, A^{∗}02:02, A^{∗}68:01, A^{∗}68:02, A^{∗}34:02, A^{∗}66:02, A^{∗}31:01:02, A^{∗}32:01, A^{∗}02:05, A^{∗}66:01, A^{∗}26:01.

For example, the HLA-A allele can include any one or more of A^{∗}02, A^{∗}11, A^{∗}24, A^{∗}30, A^{∗}33, A^{∗}03, A^{∗}01, A^{∗}26.

For example, the HLA-A allele can include any one or more of A^{∗}11:01, A^{∗}24:02, A^{∗}02:01, A^{∗}02:07, A^{∗}33:03, A^{∗}02:06 and A^{∗}30:01.

For example, the HLA-A allele can include any one or more of HLA-A^{∗}02:01:01:01, HLA-A^{∗}11:01:01:01, HLA-A^{∗}24:02:01, HLA-A^{∗}30:01:01:01, HLA-A^{∗}33:01:01:01, HLA-A^{∗}03:01:01:01, HLA-A^{∗}01:01:01:01, HLA-A^{∗}26:01:01:01.

For example, the HLA-A gene can include a nucleic acid sequence as shown in any one of SEQ ID No. 56-63.

For example, the HLA-A gene can include a nucleotide sequence that is derived from human beings and has 80% or greater, e.g., 85% or greater, 90% or greater, 95% or greater, 96% or greater, 97% or greater, 98% or greater, 99% or greater of identity as a nucleic acid sequence as shown in any one of SEQ ID No. 56-63.

### Modification

In the present application, the modification can include gene knockout and/or gene silencing.

For example, the modification can include deletion of full or partial gene fragments, gene mutations and/or gene silencing.

For example, the gene knockout can include deletion of full or partial gene fragments, gene mutations, etc.

For example, the gene can include the HLA-A gene and/or the TRAC gene.

For example, the modification can include that any one of the two TRAC alleles is knockout and any one of the two HLA-A alleles is knockout in the immune cells.

For example, the modification can include that the two TRAC alleles are knockout and any one of the two HLA-A alleles is knockout in the immune cells.

For example, the modification can include that any one of the two TRAC alleles is knockout and the two HLA-A alleles are knockout.

For example, the modification can include that the two TRAC alleles are knockout and the two HLA-A alleles are knockout.

For example, the deletion of partial gene fragments can include the deletion of ≥1 exon sequence.

For example, the gene mutations can include changes of composition or arrangement order of base pairs, which can commonly result in, e.g., frameshift mutations, missense mutations, and/or nonsense mutations, and the like. The missense mutations generally refer to the change from a codon encoding an amino acid to a codon encoding another amino acid due to the change of a certain base pair, resulting in the change of the corresponding amino acids constituting a protein. The frameshift mutations generally refer to the insertion or deletion of one or several bases that are not integral multiples of 3 in the DNA strands, resulting in the change of coding codon. The nonsense mutations generally refer to the change from a codon encoding an amino acid to a terminator due to the change of a certain base pair, causing early termination of the protein synthesis when it reaches the site.

For example, the change of composition or arrangement order of base pairs can include changes of a single nucleotide or base (also known as point mutation) and/or changes of plural nucleotides or bases. For example, the change of the single nucleotide or base can include substitution of a base or nucleotide with another base or nucleotide, and insertion or deletion of a base. For example, the change of the plural nucleotides or bases can include loss of a segment of base sequence, insertion of a segment of base sequence, and/or rearrangement of a segment of base sequence. The segment of base sequence can be a portion of any exon and/or intron in a gene. The rearrangement can include repeat, inversion, translocation of a segment of base sequence, etc.

For example, the gene silencing can include a transcriptional gene silencing (TGS) and/or a post-transcriptional gene silencing (PTGS) at a transcriptional level. The silencing at the transcriptional level can include the methylation of DNA molecules so as to suppress the DNAs, e.g., the methylation of promotor sequence. The post-transcriptional gene silencing can include a variation of gene expression caused by specifically interfering with the target RNA after gene transcription. For example, the mRNA level can be reduced by RNA interference (RNAi).

In the present application, the modification can include a technology or method including replacing the endogenous normal gene with an exogenous nucleotide sequence via homologous recombination to inactivate the endogenous normal gene. The exogenous nucleotide sequence can be well known. For example, the exogenous nucleotide sequence can be a partial fragment of the endogenous normal gene. For example, the exogenous nucleotide sequence can include, from 5' to 3', a homologous arm 1, a nucleotide sequence to be inserted, and a homologous arm 2 in sequence. The nucleotide sequence to be inserted can include a reporter gene, a noncoding sequence or a varied sequence of an endogenous normal gene.

In the present application, the modification can include administering one or more substances selected from the group consisting of antisense RNA, siRNA, shRNA, and CRISPR/Cas9 system to the immunologic effector cells.

In the present application, the antisense RNA can be a single stranded RNA complementary to the transcript mRNA (messager RNA).

For example, the complementation means that at least 60% of nucleotide sequence of the antisense RNA is complementary to the mRNA, e.g., at least 70%, e.g., at least 80%, e.g., at least 90%, e.g., 100%.

For example, the binding of the antisense RNA to a target mRNA can cause an increased sensitivity of the double stranded RNA molecule on RNA enzyme III, thereby degrading the molecule.

For example, the antisense RNA binds to the upstream noncoding region of the mRNA, thereby inhibiting the translation of the target mRNA.

For example, the antisense RNA is artificial.

For example, the antisense RNA cannot form a short hairpin structure.

In the present application, the antisense RNA includes a nucleotide sequence as shown in any one of SEQ ID NO. 93-96.

In the present application, the siRNA can be a class of double stranded and noncoding RNA molecules having about 18-28 base pairs in length.

For example, the siRNA can result in the degradation of mRNA by means of complementary binding to the mRNA.

For example, the siRNA can include 18, 19, 20, 21, 22, 23, 24, 25, 26, 27, 28 base pairs in length.

For example, the siRNA is artificial.

For example, the siRNA is generated by treating the intracellular double stranded RNA or shRNA with Dicer enzyme.

In the present application, the shRNA refers to a class of RNAs capable of forming a short hairpin structure.

For example, the shRNA can include two invert repeats, as well as a loop sequence between the two short invert repeats.

For example, the shRNA includes at least 18 continuous nucleic acid sequences that can complementarily bind to the target mRNA, e.g., at least 19, e.g., at least 20, e.g., at least 21, e.g., at least 22, e.g., at least 23, e.g., at least 24, e.g., at least 25, e.g., at least 26.

For example, the shRNA does not include the sgRNA of the present application.

In the present application, the modification can include administering CRISPR/Cas system to the immunologic effector cells.

### CRISPR/Cas system

In the present application, the CRISPR/Cas system can include a guide RNA (gRNA) and Cas enzyme. The gRNA can include crRNA and tracrRNA. When the crRNA and the tracrRNA are located in two different nucleotide molecules, respectively, the gRNA can be called dgRNA (bimolecular gRNA). When the crRNA and the tracrRNA are located in the same nucleotide molecule, the gRNA can be called sgRNA (unimolecular gRNA).

In the present application, the CRISPR/Cas system can include a gRNA nucleic acid sequence and a Cas protein. For instance, the gRNA nucleic acid sequence and the Cas protein form an RNP complex.

In the present application, the CRISPR/Cas system can further include a nucleic acid sequence encoding the gRNA and a nucleic acid sequence encoding Cas protein. The nucleic acid sequence encoding the gRNA and the nucleic acid sequence encoding the Cas protein can be disposed in vectors that are commonly used in the art, e.g., plasmids, viruses (e.g., adenoviruses, lentiviruses, retroviruses), or the like. For example, the nucleic acid sequence encoding the gRNA and the nucleic acid sequence encoding the Cas protein can be located in the same or different vectors. For example, the nucleic acid sequence encoding the tracrRNA and the sequence encoding the crRNA can be located in the same or different vectors. Promotors for driving the expression of various coding sequences can be the same or different.

In the present application, the CRISPR/Cas system can include more than one guide RNA. Each guide RNA can include a different targeting sequence, so that the CRISPR/Cas system cleaves more than one target sequence. For example, one or more guide RNAs can include the same or different characteristics, e.g., activity or stability in the CRISPR/Cas complex. If more than one guide RNAs are used, each guide RNA can be encoded in the same or different vectors. Promotors for driving the expression of the more than one guide RNAs can be the same or different. For example, the guide RNA includes those targeting the HLA-A gene or targeting the TRAC gene.

In the present application, the modification further includes administering a Cas enzyme to the cell. The Cas enzyme can include a Cas protein, and a nucleic acid sequence encoding the Cas protein.

For example, the Cas protein can include at least one domain that interacts with the guide RNA (gRNA). For example, the Cas protein can be directed by the guide RNA to a target sequence. For example, the guide RNA interacts with the Cas protein and the target sequence, thereby directing the binding of the Cas protein to the target sequence. For example, the guide RNA provides a specificity to the target cleaving, wherein the Cas protein can be universal and is paired with various guide RNAs to cleave different target sequences. For example, the Cas protein can cleave a single or double stranded DNA. For example, the Cas protein can cleave RNA. For example, the Cas protein can lead to an incision occurred in the RNA/DNA. For example, the Cas protein includes at least one DNA binding domain and at least one nuclease domain. For example, the nuclease can be exogenous in relation to the DNA binding domain; e.g., the nuclease activity can be changed by modifying the Cas protein. For example, the Cas protein can be used for binding to the DNA and regulating the expression or activity of the DNA. For example, the Cas protein can be a Cas nuclease.

In the present application, the CRISPR/Cas system can include Class 1 or Class 2 system components, including a ribonucleotide-protein complex (see, e.g., Makarova et al., Nat Rev Microbiol, 13(11):722-36(2015); Shmakov et al., Molecular Cell,60:385-397(2015). Of those, Class 2 CRISPR/Cas system has a simple proteineous effector. Type II, V and VI Cas proteins can be a simple protein, an RNA guide nuclease, which are called "Class 2 Cas nuclease" in the present application. Class 2 Cas nuclease can include Cas9, Cpfl, C2c1, C2c2 and C2c3 proteins. Cas9 or Cpfl protein (Zetsche et al., Cell,163:1-13(2015)) can include an RuvC-like nuclease domain or an HNH-like nuclease domain, the Cpfl sequence in Zetsche is incorporated herein by reference in its entirety.

For example, Cas protein can be derived from Type II CRISPR/Cas system (that is, the Cas9 protein of the CRISPR/Cas9 system) or Type V CRISPR/Cas system (e.g., Cpfl protein). For example, the Cas protein can be derived from Class 2 CRISPR/Cas system, e.g., Cas9 protein or Cpfl protein. Proteins of Class 2 Cas nuclease family are enzymes with DNA endonuclease activity, which can be directed to cleave the desired nucleic acid target by designing an appropriate guide RNA as further described herein.

For example, the components of Class 2 CRISPR/Cas system can be derived from Type IIA, IIB, IIC, V or VI system, including Cas9 and its orthologs. Cas9 protein or its orthologs can be derived from exemplary species of Streptococcus pyogenes, Streptococcus thermophilus, Streptococcus sp., Staphylococcus aureus, Listeria in-nocua, Lactobacillus gasseri, Francisella novicida, Wolinella succinogenes, Neisseria meningitidis, Campylobacter jejuni, Pasteurella multocida, Fibrobacter succinogene, Rhodospirillum rubrum, Nocardiopsis dassonvillei, Streptomycespristinaespiralis, Streptomyces viridochromogenes, Streptosporangium roseum, Alicyclobacillusacidocaldarius, Bacillus pseudomycoides, Lactobacillusdelbrueckii, Lactobacillus salivarius, Lactobacillusbuchneri, Treponema denticola, Microscilla mari-na, Burkholderiales bacterium, Polaromonas naphthalenivorans, Polaromonas sp., Cyanothecesp., Microcystis aeruginosa, Synechococcus sp., Acetohalobium arabati-cum, Clostridium botulinum, Clostridium difficile, Marinobacter sp., Nitrosococcus halophilus, Anabaena variabilis, Nodularia spumigena, Nostoc sp., Arthrospiramaxima, Arthrospira platensis, Arthrospirasp., Lyngbya sp., Microcoleuschthonoplastes, Oscillatoria sp., Thermosipho africanus, Neisseriacinereal, Campylobacter lari, or Corynebacterium diphtheria.

For example, the Cas9 protein can be derived from Streptococcus pyogenes. For example, the Cas9 protein can be derived from Streptococcus thermophilus. For example, the Cas9 protein can be derived from Staphylococcus aureus. For example, the Cpfl protein can be derived from Francisella tularensis, Acidaminococcus, Eubacterium eligens, Leptospira inadai, Prevotella dis-iens or Porphyromonasmacacae. For example, the Cpfl protein can be derived from Acidaminococcus or Lachnospiraceae.

For example, the Cas9 protein can include an amino acid sequence having 60%, 65%, 70%, 75%, 80%, 85%, 90%, 95%, 96%, 97%, 98%, or 99% of identity with Streptococcus pyogenes Cas9.

For example, the Cas9 protein can include an amino acid sequence as shown in SEQ ID NO: 65.

For example, the exemplary nucleic acid sequences encoding Cas9 protein are described in the following literatures: Cong et al., SCIENCE 2013,399(6121):819-823; Wang et al., CELL 2013,153(4):910-918; Mali et al., SCIENCE 2013,399(6121):823-826; Jinek et al., SCIENCE 2012,337(6096):816-821.

For example, the nucleotide sequence encoding Cas9 protein is as shown in SEQ ID NO: 64.

For example, the Cas9 protein can be modified. For example, the modification may include amino acid substitution and forming a fusion protein with other polypeptide fragments. The other polypeptide fragments can include PEST sequences, ubiquitin, polyubiquitin, and nuclear localization signal (NLS).

In the present application, the crRNA can include a targeting sequence, by which the gRNA can target any target sequence. For example, the complementary degree between the targeting sequence and the targeting sequence of the target nucleic acid molecule can be about 60%, 65%, 70%, 75%, 80%, 85%, 90%, 95%, 97%, 98%, 99% or 100%. For example, the targeting sequence can be 100% complementary to the targeting sequence of the target nucleic acid molecule. For example, the targeting sequence and the targeting sequence of the target nucleic acid molecule can include at least one mispairing. For example, the targeting sequence and the targeting sequence of the target nucleic acid molecule can include 1, 2, 3, 4, 5, 6, 7, 8, 9 or 10 mispairings. For example, the targeting sequence and the targeting sequence of the target nucleic acid molecule can include 1-6 mispairings. For example, the targeting sequence and the targeting sequence of the target nucleic acid molecule can include 5 or 6 mispairings. For example, the targeting sequence and the targeting sequence of the target nucleic acid molecule do not include a mispairing.

In the present application, the length of the targeting sequence can depend on the used CRISPR/Cas system and components. For example, different Cas proteins derived from different bacteria species have different optimal targeting sequence length. Moreover, the targeting sequence can include 5, 6, 7, 8, 9, 10, 11, 12, 13, 14, 15, 16, 17, 18, 19, 20, 21, 22, 23, 24, 25, 26, 27, 28, 29, 30, 35, 40, 45, 50 or more than 50 nucleotides in length. For example, the targeting sequence can include 18-24 nucleotides in length. For example, the targeting sequence can include 19-21 nucleotides in length. For example, the targeting sequence can include 20 nucleotides in length.

In the present application, the crRNA can further include a crRNA flagpole sequence including any sequence that can be complementary to the tracrRNA so as to facilitate the formation of a CRISPR/Cas complex. For example, the flagpole sequence can include a full or a portion of sequence of naturally occurring crRNA that is complementary to the tracrRNA in the same CRISPR/Cas system (also known as "label" or "handle"). For example, the flagpole sequence can include a full or a portion of repeat sequence from naturally occurring CRISPR/Cas system. For example, the flagpole sequence can include a truncated or modified label or a handle sequence. The portion in the tracrRNA complementary to the crRNA flagpole sequence can be called tracrRNA flagpole sequence. For example, the complementary degree between the tracrRNA and the flagpole portion hybridized with the tracrRNA along the shorter of the two sequences can be about 40%, 50%, 60%, 70%, 80% or higher. For example, the tracrRNA and the flagpole portion hybridized with the tracrRNA along the shorter of the two sequences are not 100% complementary. The length of the crRNA flagpole sequence can depend on the used CRISPR/Cas system or tracrRNA. For example, the crRNA flagpole sequence can include 10-50 nucleotides or more than 50 nucleotides in length. For example, the crRNA flagpole sequence can include 15-40 nucleotides in length. For example, the crRNA flagpole sequence can include 20-30 nucleotides in length. For example, the crRNA flagpole sequence can include 22 nucleotides in length. When a dual-guide RNAis used, there can be no upper limit in the crRNA flagpole sequence length.

In the present application, the tracrRNA can include a full or a portion of wild-type tracrRNA sequence derived from naturally occurring CRISPR/Cas system. For example, tracrRNA can include a truncated or modified variant of wild-type tracrRNA. The length of the tracrRNA can depend on the used CRISPR/Cas system. For example, the tracr RNA can include 5, 6, 7, 8, 9, 10, 11, 12, 13, 14, 15, 16, 17, 18, 19, 20, 25, 30, 40, 50, 60, 70, 80, 90, 100 or more than 100 nucleotides in length. For example, the length of tracr is at least 20 nucleotides. For example, the length of tracrRNA is at least 40 nucleotides. For example, the tracrRNA can include a secondary structure, e.g., one or more hairpin or stem-loop structures, or one or more convex structures.

In the present application, the guide RNA can include two RNA molecules, and called "dual-guide RNA" or "dgRNA" herein. For example, the dgRNA can include a first RNA molecule containing a crRNA and a second RNA molecule containing a tracrRNA. The first and the second RNA molecules can form an RNA duplex by base pairing between the flagpole sequences on the crRNA and the tracr RNA.

For example, the first RNA molecule can include, from 5' to 3', a targeting sequence complementary to a target sequence, and a crRNA flagpole sequence. The second RNA molecule can include, from 5' to 3', a tracrRNA flagpole sequence complementary to a crRNA flagpole sequence, and a nuclease binding sequence. For instance, the nuclease binding sequence can be a Cas nuclease, such as, Cas9.

In the present application, the guide RNA can include a single RNA molecule, called "single molecular gRNA" or "sgRNA". For example, the sgRNA can include a tracrRNA, a crRNA covalently linked to the tracrRNA. For example, the crRNA and the tracrRNA can be covalently linked through a linker nucleic acid sequence. For example, the single molecular gRNA can include a loop-stem structure formed by base pairing between the flagpole sequences on the crRNA and the tracr RNA. For example, the sgRNA is a "Cas9sgRNA" that can mediate a DNA cleavage by means of Cas9 protein. For example, the sgRNA is a "Cpf1sgRNA" that can mediate a DNA cleavage by means of Cpfl protein.

For example, the single molecular gRNA or sgRNA can include, from 5' to 3', a crRNA, a loop, and a tracrRNA. The crRNA can include, from 5' to 3', a targeting sequence complementary to the target sequence, and a crRNA flagpole sequence. The tracrRNA can include, from 5' to 3', a tracrRNA flagpole sequence complementary to a crRNA flagpole sequence, and a nuclease binding sequence. For example, the nuclease binding sequence can be a Cas nuclease. For example, Cas9.

For example, the single molecular gRNA or sgRNA can include, from 5' to 3', a targeting sequence complementary to the target sequence, a crRNA flagpole sequence, a loop, a tracrRNA flagpole sequence complementary to a crRNA flagpole sequence, and a nuclease binding sequence.

In the present application, the gRNA can further include modified nucleosides or nucleotides. For example, the modification can be changing (e.g., replacing) one or two non-linker phosphate oxygens and/or one or more linker phosphate oxygens in the phosphate diester bond, e.g., changing (e.g., replacing) the components of ribose, e.g., replacing the 2 'hydroxyl group on ribose; e.g., replacing the phosphate moiety with a dephosphorization linker; e.g., modification or replacement of naturally occurring nuclear bases; e.g., replacing or modifying the backbone of ribose phosphate; e.g., modification of the 3 'or 5' end of an oligonucleotide, such as deleting, modifying, or replacing a terminal phosphate group or a conjugated portion, capping or splicing (e.g., 3 'or 5' capping may include sugar and/or backbone modifications); such as, modifying or replacing sugars.

For example, introducing the modified nucleosides or nucleotides increases the stability to the nuclease. For example, introducing the modified nucleoside or nucleotide reduces the congenital immune reaction. The congenital reactions include a cellular reaction against an exogenous nucleic acid (including single strand nucleic acids), that can involve inducing the expression and release of cytokines (especially interferons) and inducing death of cells.

For instance, the modification can include administering an sgRNA targeting an exon of the HLA-A gene to the immunologic effector cells.

For instance, the sgRNA targeting the exon of the HLA-A gene can include a nucleotide sequence shown in any one of SEQ ID No. 16-54 and 91-92.

For instance, the sgRNA targeting the exon portion of the HLA-A gene can include a nucleotide sequence having at least 70% of identity with a nucleotide sequence as shown in any one of SEQ ID No: 16-54 and 91-92, such as, at least 75%, 80%, 85%, 90%, 95%, 96%, 97%, 98%, and 99%.

For example, the modification can include administering an sgRNA targeting an exon of the TRAC gene to the immunologic effector cells.

For example, the sgRNA targeting the exon of the TRAC gene can include a nucleotide sequence shown in any one of SEQ ID No. 1-15.

For example, the sgRNA targeting the exon of the TRAC gene can include a nucleotide sequence having at least 70% with a nucleotide sequence as shown in any one of SEQ ID No. 1-15, e.g., at least 75%, 80%, 85%, 90%, 95%, 96%, 97%, 98%, and 99%.

### Chimeric Antigen Receptor (CAR)

In the present application, the immunologic effector cells include a nucleic acid encoding a chimeric antigen receptor (CAR), and the CAR includes an antigen binding domain, a hinge region, a transmembrane domain, a co-stimulating structure, and a primary signal transduction domain.

For example, the antigen binding domain specifically binds to a tumor antigen. For example, the tumor antigen is selected from the group consisting of CD19, CD123, CD22, CD30, CD171, CA125, C-met, L1CAM, EC, DLL3, CD99, 5T4, CD138, CS-1 (also known as CD2 subclass 1, CRACC, SLAMF7, CD319 or 19A24), C-type lectin-like molecule-1 (CLL-1 or CLECL1), CD33, epidermal growth factor receptor variant III (EGFRvIII), ganglioside G2 (GD2), ganglioside GD3, TNF receptor family member B cell mature antigen (BCMA), Tn antigen (e.g., Tn Ag, GalNAcα-Ser/Thr), prostate-specific membrane antigen (PSMA); receptor tyrosine kinase-like orphan receptor 1 (ROR1), Fms-like tyrosine kinase 3 (FLT3); tumor-associated glycoprotein 72 (TAG72), CD38, CD44v6, carcino-embryonic antigen (CEA), epithelial cell adhesion molecule (EPCAM), B7H3 (CD276), KIT (CD117), interleukin-13 receptor subunit α-2 (IL-13Ra2 or CD213A2), mesothelin, interleukin 11 receptor α (IL-11Ra), prostate stem cell antigen (PSCA), protease serine 21, vascular endothelial growth factor receptor 2 (VEGFR2), Lewis (Y) antigen, CD24, platelet-derived growth factor receptor β (PDGFR-β), stage-specific embryonic antigen-4 (SSEA-4), CD20, folate receptor α, receptor tyrosine-protein kinase ERBB2 (Her2/neu), cell surface-associated mucoprotein 1 (MUC1), epidermal growth factor receptor (EGFR), neurocyte adhesion molecule (NCAM), Prostase, prostate acid phosphatase (PAP), elongation factor 2 mutant (ELF2M), ephrin B2, fibroblast activated protein α (FAP), insulin-like growth factor 1 receptor (IGF-I receptor), carbonic anhydrase IX (CAIX), proteasome (e.g., proteasome, giantin factor) subunit B-type 9 (LMP2), glycoprotein 100 (gp100), oncogene fusion protein (bcr-abl) composed of breakpoint cluster region (BCR) and Abelson murine leukemia virus oncogene homolog 1 (Abl), tyrosinase, ephrin A-type receptor 2 (EphA2), fucosyl GM1; sialyl Lewis adhesion molecule (sLe), transglutaminase 5 (TGS5), high molecular weight-melanoma-associated antigen (HMWMAA), o-acetyl-GD2 ganglioside (OAcGD2), folate receptor β, tumor endothelial marker 1 (TEM1/CD248), tumor endothelial marker 7-associated (TEM7R), tight junction protein 6 (CLDN6), thyrotropin receptor (TSHR), G-protein-coupled receptor C-class Group 5 Member D (GPRC5D), chromosome X ORF 61 (CXORF61), CD97, CD179a, anaplastic lymphoma kinase (ALK), polysialic acid, placental specificity 1 (PLAC1), hexose moiety of globoH glycoceramide (GloboH), mammary gland differentiation antigen (NY-BR-1), uroplakin 2 (UPK2), hepatitis virus A cell receptor 1 (HAVCR1), adrenaline receptor β3 (ADRB3), pannexin 3 (PANX3), G-protein-coupled receptor 20 (GPR20), lymphocyte antigen 6 complex loci K9 (LY6K), olfactory receptor 51E2 (OR51E2), TCRγ variable ORF protein (TARP), Wilm tumor protein (WT1); cancer/testis antigen 1 (NY-ESO-1), cancer/testis antigen 2 (LAGE-1a), melanoma-associated antigen 1 (MAGE-A1), ETS translocation variant gene 6 located on chromosome 12p (ETV6-AML), sperm protein 17 (SPA17), X antigen family member 1A (XAGE1), angiogenin cell surface receptor 2 (Tie 2), melanoma cancer/testis antigen-1 (MAD-CT-1), melanoma cancer/testis antigen-2 (MAD-CT-2), Fos-associated antigen 1, p53, p53 mutant, prostate-specific protein (prostein), prostate cancer tumor antigen-1 (PCTA-1 or galectin 8), T cell-recognized melanoma antigen 1 (MelanA or MARTI); rat sarcoma (Ras) mutant, human telomerase reverse transcriptase (hTERT), sarcoma translocation breakpoint, melanoma anti-apoptosis protein (ML-IAP), ERG (transmembrane protease, serine 2 (TMPRSS2) ETS fusion gene), N-acetylglucosaminyltransferase V (NA17), paired frame protein Pax-3 (PAX3), androgen receptor, cyclin B1, v-myc avian myelocytosis virus oncogene neuroblastoma-derived homologs (MYCN), Ras homolog family member C (RhoC), tyrosinase-associated protein 2 (TRP-2), cytochrome P450 1B1 (CYP1B1), T cell-recognized squamous cell cancer antigen 3 (SART3), paired frame protein Pax-5 (PAX5), proacrosomal protein binding protein sp32 (OY-TES1), lymphocyte-specific protein tyrosine kinase (LCK), A kinase anchorin 4 (AKAP-4), synovial sarcoma X breakpoint 2 (SSX2), receptor of advanced glycation end products (RAGE-1), legumain, human papilloma virus E6 (HPV E6), human papilloma virus E7 (HPV E7), intestinal carboxylesterase, mutated heat shock protein 70-2 (mut hsp70-2), CD79a, CD79b, CD72, leukocyte-associated immunoglobulin-like receptor 1 (LAIR1), Fc fragment of IgAreceptor (FCAR or CD890), leukocyte immunoglobin-like receptor superfamily A member 2 (LILRA2), CD300 molecule-like family member f (CD300LF), C-type lectin domain family 12 member A (CLEC12A), bone marrow stromal cell antigen 2 (BST2), EGF-like modulus-containing mucoprotein-like hormone receptor-like 2 (EMR2), lymphocyte antigen 75 (LY75), glypican-3 (GPC3), Fc receptor-like 5 (FCRL5) and/or immunoglobulin λ-like peptide 1 (IGLL1).

In the present application, the antigen binding domain can include an antibody specifically binding to the tumor antigen or a fragment of antigen binding thereof. For example, the antibody specifically binding to GPC3 or a fragment of antigen binding thereof as described in the present application can include, but is not limited to, recombinant antibody, monoclonal antibody, human antibody, humanized antibody, chimeric antibody, bispecific antibody, single stranded antibody, duplex antibody, triplex antibody, quadruplex antibody, Fv fragment, scFv fragment, Fab fragment, Fab' fragment, F (ab') 2 fragment and Camelidae single domain antibody.

For example, the antibody can be a humanized antibody. It can be an antibody that immuno-specifically binds to an associated antigen (e.g., human CD19, BCMA or GPC3) and contains a framework (FR) region having substantially an amino acid sequence of human antibody and a complementary determining region (CDR) having substantially an amino acid sequence of nonhuman antibody, or its variant, derivative, analogue, or fragment. As used herein, "substantially" with reference to the CDR means that the amino acid sequence of the CDR has at least 80%, at least 85%, at least 90%, at least 95%, at least 98% or at least 99% of identity with an amino acid sequence of nonhuman antibody CDR.

For example, the antigen binding fragment can include Fab, Fab', F (ab) 2, Fv fragment, F (ab') 2, scFv, di-scFv and/or dAb.

For example, the single stranded antibody is scFv.

For example, the antigen binding domain targets a solid tumor. For example, the solid tumor is selected from the group consisting of: liver cancer, stomach cancer, lung cancer, breast cancer, colon cancer, rectal cancer, renal cell carcinoma, liver cancer, non-small cell lung cancer, small intestine cancer, esophagus cancer, melanoma, osteosarcoma, pancreatic cancer, skin cancer, head or neck cancer, cutaneous or intraocular malignant melanoma, uterus cancer, ovarian cancer, rectal cancer, testis cancer, fallopian tube carcinoma, endometrial cancer, cervical cancer, vaginal cancer, vulval cancer, Hodgkin's disease, non-Hodgkin's lymphoma, carcinoma of endocrine system, thyroid cancer, parathyroid cancer, adrenal carcinoma, soft tissue sarcoma, urethral carcinoma, carcinoma of penis, pediatric solid tumor, bladder cancer, renal or ureteral cancer, carcinoma of renal pelvis, central nervous system (CNS) tumor, primary CNS lymphoma, tumor angiogenesis, spinal tumor, brainstem glioma, pituitary adenoma, Kaposi sarcoma, epidermoid, squamous cell carcinoma, T cell lymphoma, and metastatic lesions of these cancers.

For example, the antigen binding domain targets a non-solid tumor. For example, the non-solid tumor is selected from the group consisting of chronic lymphoblastic leukemia (CLL), acute leukemia, acute lymphoblastic leukemia (ALL), B cell acute lymphoblastic leukemia (B-ALL), T cell acute lymphoblastic leukemia (T-ALL), chronic myeloid leukemia (CML), acute myeloid leukemia (AML), B cell prolymphocytic leukemia, blast cell plasmacytoid dendritic cytoma, Burkitt's lymphoma, diffuse large B cell lymphoma, follicular lymphoma, hairy cell leukemia, small or large cell follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablast lymphoma, plasmacytoid dendritic cytoma, and Waldenstrom macroglobulinemia.

In the present application, the transmembrane domain can include a transmembrane domain of protein selected from the group consisting of CD28, CD3e, CD45, CD4, CD5, CD8, CD9, CD16, CD22, CD33, CD37, CD64, CD80, CD86, CD134, CD137 and CD154.

In the present application, the costimulatory domain can include a costimulatory domain of protein selected from the group consisting of CD137, CD28, 4-1BB, OX40 and ICOS.

In the present application, the intracellular signal transduction domain can include a signal transduction domain derived from CD3ζ.

In the present application, the hinge region connects the antigen binding domain with the transmembrane domain, and includes a hinge region of protein selected from the group consisting of IgG1, IgG4, IgD, and CD8.

### Composition, Use

The present application provides a composition including the immunologic effector cells of the present application and a pharmaceutically acceptable carrier.

For example, the composition includes a cell population, wherein the cell population includes the modified immunologic effector cell of the present application.

For example, the ratio of the modified immune effector cells to the total cells in the cell population is at least 0.001%, at least 0.01%, at least 0.1%, at least 1%, at least 5%, at least 10%, at least 15%, at least 20%, at least 25%, at least 30%, at least 35%, at least 40%, at least 45%, at least 50%, at least 53%, at least 55%, at least 58%, at least 60%, at least 63%, at least 65%, at least 68%, at least 70%, at least 73%, at least 75%, at least 78%, at least 80%, at least 83%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%.

For example, the cell population can include the modified immune effector cells and the corresponding unmodified immune effector cells.

For example, the modified immune effector cells can include cells in which any one of the two TRAC alleles is knockout and any one of the two HLA-A alleles is knockout, cells in which both of the two TRAC allele are knockout and any one of the two HLA-A alleles is knockout, cells in which any one of the two TRAC alleles is knockout and both of the two HLA-A alleles are knockout, and cells in which both of the two TRAC alleles are knockout and both of the two HLA-A alleles are knockout.

For example, the cell population can be a cell population obtained by treating a population of the immune effector cells by means of genetic engineering, wherein the means of genetic engineering include administering the antisense RNA, the siRNA, the shRNA and/or CRISPR/Cas9 system to the cell population of the immune effector cells. For example, the CRISPR/Cas9 system can include the sgRNA targeting the exon of the HLA-A gene, the sgRNA targeting the exon of the TRAC gene, and the Cas9 protein.

For example, the cell population can include a cell population obtained by editing a cell population of the immune effector cells with CRISPR/Cas9 system with an editing efficiency of at least 30%, at least 35%, at least 40%, at least 45%, at least 50%, at least 53%, at least 55%, at least 58%, at least 60%, at least 63%, at least 65%, at least 68%, at least 70%, at least 73%, at least 75%, at least 78%, at least 80%, at least 83%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%.

For example, the editing efficiency can be obtained by Sanger sequencing, TA clone sequencing, and flow cytometry.

For example, the cell population can include a cell population obtained by administering the antisense RNA, the siRNA, the shRNA of the present application to the cell population of the immune effector cells, wherein the mRNA expression in the cell population is reduced by at least 10% at least 20%, at least 30%, at least 35%, at least 40%, at least 45%, at least 50%, at least 53%, at least 55%, at least 58%, at least 60%, at least 63%, at least 65%, at least 68%, at least 70%, at least 73%, at least 75%, at least 78%, at least 80%, at least 83%, at least 85%, at least 86%, at least 87%, at least 88%, at least 89%, at least 90%, as compared with that in the cell population of the corresponding immune effector cells prior to the administration.

For example, the cell population can include a cell population obtained by administering the antisense RNA, the siRNA, the shRNA of the present application to the cell population of the immune effector cells, wherein the expression of protein in the cell population is reduced by at least 10%, at least 20%, at least 30%, at least 35%, at least 40%, at least 45%, at least 50%, at least 53%, at least 55%, at least 58%, at least 60%, at least 63%, at least 65%, at least 68%, at least 70%, at least 73%, at least 75%, at least 78%, at least 80%, at least 83%, at least 85%, at least 86%, at least 87%, at least 88%, at least 89%, at least 90%, as compared with that in the cell population of the immune effector cells prior to the administration.

For example, the acceptable ingredients in the composition are nontoxic to the recipient at the used dose and concentration. The pharmaceutical composition of the present invention includes, but is not limited to, liquid, frozen, and lyophilized compositions.

For example, the pharmaceutically acceptable carrier can include any and all solvents, dispersing mediums, isotonic agents, and absorption retarder that are compatible with the immune effector cells, which may be usually safe and nontoxic, and neither biologically nor otherwise undesired. For example, the pharmaceutically acceptable carrier can include a storage liquid, a cryopreservation liquid, injectable solution at 2°C-8°C, etc. For instance, the carrier can include the following ingredients: adenosines, sodium chloride, albumin, interleukin-15, angiotensin-II, short peptides and polypeptide compounds in serum-free medium of human umbilical cord mesenchymal stem cells. For example, the carrier can further include Normosol R (Abbott), Plasma-Lyte A (Baxter) injectable solution, 5% aqueous glucose solution or Ringer's lactate solution. For example, the carrier can further include glycine or DMSO.

For example, the composition can include parenteral, transdermal, endoluminal, intra-arterial, intrathecal and/or intranasal administration or direct injection into tissue. For example, the composition can be administered to a patient or a subject via infusion or injection. In some embodiments, the pharmaceutical composition can be administered in different manners, e.g., administered intravenously, intraperitoneally, subcutaneously, intramuscularly, locally or dermally. In some embodiments, the pharmaceutical composition can be administered in an uninterrupted method. The uninterrupted (or continuous) administration can be achieved by a small pump system worn by the patient to measure therapeutic agent flowing into the patient, as described in WO2015/036583.

The present application further provides use of the modified immunologic effector cells of the present application for preparing a CAR-T cell.

The present application further provides use of the modified immunologic effector cells of the present application in manufacture of a drug for allogeneic therapy.

The present application further provides use of the modified immunologic effector cells of the present application in manufacture of a drug for treating tumors.

For example, the tumor includes solid and non-solid tumors. The solid tumors and the non-solid tumors are as described above.

Without being limited by any theory, the following examples are only for illustrating the modified immunologic effector cells, the preparation method and use of the present application, and are not intended to limit the scope of the invention of the present application. The examples do not include a detailed description of traditional methods, such as, methods used for constructing vectors and plasmids, methods of inserting a gene encoding the protein into such vectors and plasmids, or methods of introducing the plasmid into a host cell. Such methods are well known for persons with ordinary skills in the art, and have been described in a plurality of publications, including Sambrook, J., Fritsch, E. F. andManiais, T. (1989) Molecular Cloning: A Laboratory Manual, 2nd edition, Cold spring Harbor Labora-tory Press.

### EXAMPLES

### EXAMPLE 1 Design of guide RNA

On the website https://www.ncbi.nlm.nih.gov/, the corresponding gene sequences (as shown in SEQ ID No.55-63) were searched and downloaded. The gene sequences were opened with Snap Gene software, and sgRNAs could be designed on different exons of the target gene. The sgRNA of CRISPR/Cas9 system used in this example was designed following a non-restrictive principle of 5'- NNN (20)- NGG-3', wherein NGG was called primary spacer adjacent motif (PAM), wherein N represented A, T, C, or G. Since many sgRNAs could be designed on the same exon, and the sgRNA composed of 20 nucleotide sequences may repeatedly occur in the genome, the design and evaluation of sgRNAs were carried out on the website http://crispr.cos.uni-heidelberg.de. An exon sequence was copied into the website, and then the website designed sgRNAs and performed a prediction evaluation. The higher the evaluation score, the higher the editing efficiency and the lower the off-target risk. The sgRNA with higher score was selected for detection. The sgRNAs targeting the TRAC gene were as shown in SEQ ID No. 1-15; the sgRNAs targeting the HLA-A02 gene were as shown in SEQ ID No. 16-37; the sgRNAs targeting the HLA-A11 gene were as shown in SEQ ID No. 38-46; and the sgRNAs targeting the HLA-A24 gene were as shown in SEQ ID No. 47-54, which were synthesized by Kingsley Biotech.

### EXAMPLE 2 Preparation of CD3⁺ T Cells

### (1) Isolation of PBMCs from peripheral blood

Peripheral blood was collected from healthy volunteer, and diluted with PBS buffer at a ratio of 1: 1. To a new 50 ml centrifuge tube was first added 1/3 of the diluted blood volume of cell separation solution (Ficoll). Then, a dilution of blood cells was added very slowly along the tube wall. The mixture was subject to centrifugation at 800g at room temperature for 20 min (the centrifuge was set at speed up 1, speed down 0). After centrifugation, the liquid in the centrifuge tube was separated to PBS and serum layer, white blood cell layer, lymphocyte separation fluid and red blood cell layer from top to bottom. The PBS and serum layer were removed; the white blood cell layer was transferred to a new 50ml centrifuge tube, and PBS was added to a volume of 40ml to wash the cells. The mixture was subject to centrifugation at 450g for 10min. After centrifugation, the supernatant was discarded to obtain peripheral blood mononuclear cells. After resuspension, the cells were counted.

### (2) Thawing of cryopreserved healthy human PBMCs

The cryopreserved PBMC cells were thawn in a 37 °C water bath. After complete thawing, the cells were sucked into a 15 ml centrifuge tube containing 10 ml X-VIVO15 medium containing 10% FBS (purchased from LONZA) and centrifuged at 400 g for 8 min. The supernatant was discarded, and 2ml X-VIVO15 medium (containing 10% FBS and a 100µg/m final concentration of DNase I) was added and incubated at room temperature for 15 min under constant shaking. After incubation, the solution was filtered with 40µm filter mesh. The cells at the bottom were resuspended in 10 ml PBS buffer, and added onto a filter mesh. After filtration, the filtrate was subject to centrifugation at 400g for 8 min. After centrifugation, the supernatant was discarded, and the cells were resuspended and counted.

### (3) Sorting of CD3⁺T cells

EasySep^{™} Human T cell sorting kit (purchased from StemCell Technologies, Item No.: 17951) was used to extract T cells in the peripheral blood mononuclear cells (PBMCs). PBMCs were adjusted to a density of 5×10⁷ cell/ml with a PBS buffer in a range of 0.25-2ml. First, a cocktail was added and uniformly mixed, and then an isolation cocktail was added at 50µl/ml. After uniform mixing, the mixture stood at room temperature for 5min. The RapidSpheres were whirled with a vortex oscillator for 30 seconds, added into the cells at 40µl/ml, and mixed well. The mixture was supplemented with a buffer to the fold of 2.5ml, and gently blown up and down for 2-3 times. The mixture was added into cryopreservation tubes with 2.5ml in each tube. The cryopreservation tubes were placed on a magnetic frame at room temperature for 3min. The covers of the cryopreservation tubes were gently removed, and the magnetic frames were carefully picked up by holding both ends thereof, and inverted for 2-3 seconds. The cell liquid was poured into a new centrifugation tube at one time. The cells were resuspended in 10-20 ml buffer (depending on the cell counts), and subject to centrifugation at 300g for 10min. The supernatant was discarded to obtain CD3⁺T cells.

### (4) Activation of T cells

An activation reagent was formulated according to the volume ratio of medium: Transact = 99:1. The culture medium was X-VIVO15 medium (containing 5% FBS, 200U/ml IL2, 10 ng/ml IL7 and 5 ng/ml IL15), and Transact was purchased from Meitianni. T cells were thoroughly resuspended with 1 ml activation reagent (containing 10 µl Transact) per 1×10⁶ cells, and then incubated in 37°C, 5%CO2 incubator for 3 days.

### EXAMPLE 3 Preparation of single knockout cells

RNP complexes were transferred into activated T cells prepared in Example 2 by electroporation using an electroporation Kit (purchased from LONZA, Item No. V4XXP-3024). The medium (X-VIVO15 medium + 10% FBS + IL2 (200 U/ ml) + IL7 (10 ng/ml) + IL15 (5 ng/ml)) was preheated for 30 min in advance. The electrokinetic buffer was formulated at a ratio of Nucleofector Solution: Supplement =82:18. Preparation of RNP complex: the sgRNA sequence of TRAC was sg9 (as shown in SEQ ID No. 1), the sgRNA sequence of HLA-A is HLA-A02 Sg2 (as shown in SEQ ID No. 17) or HLA-A02 Sg5 (as shown in SEQ ID No. 18) or HLA-A11 sg21 (as shown in SEQ ID No. 91) or HLA-A11 Rsg2 (as shown in SEQ ID No. 92). 20 µg sgRNA was first added into a PCR tube (RNA enzyme free), and then 10 µg Cas9 protein (purchased from thermo, Item No. A36499) was added. After mixing well, the mixture was incubated for 12 min. The activated T cells cultured in Example 2 were counted, and subject to centrifugation at 300 g for 8 min. The supernatant was discarded, and PBS was added to resuspend the cells. 1E7 cells were taken for additional centrifugation at 300 g for 8 min. The supernatant was discarded, and the cells were resuspended in 100 µl of the prepared electroporation buffer. The incubated RNP complexes were added to the above cell suspension, mixed gently, and then the mixture was gently transferred to the electrofection cup. The electrofection cup was placed on the Lonza-4D electric rotating instrument, and the EO-115 electric rotating program was selected for electric rotating. Pre-heated medium was added into the electrofection cup, and then the cells were transferred into the preheated medium in the well plate with a supporting pipette, and then cultured in a 37 °C, 5% CO₂ incubator.

### EXAMPLE 4 Comparison of methods for detecting the gene knockout efficiency

### (1) Detection of Sanger sequencing

The cells were counted, and 3∼5 ×10⁴cells were subject to centrifugation at 2000r/min for 5min. The supernatant was discarded as much as possible, and 20µl DE lysing solution was added onto each tube. Lysed cells were added into the PCR tube, centrifuged instantaneously and then placed into the PCR apparatus with the following conditions: 65 °C for 30 min, 4 °C for 30 s, 95 °C for 2 min and 16 °C for infinite time. PCR was performed by using primer pairs TRAC-For/TRAC-Rev or HLA-A For/HLA-A Rev with the cleavage products as templates. The sequence of PCR primers was as shown in SEQ ID NO.66-81. The PCR products were sent to Jin Weizhi for Sanger sequencing. After getting the Sanger sequencing results, the editing site and the editing efficiency were predicted with the EditR editor on the website: https://moriaritylab.shinyapps.io/editr_v10/.

### (2) Detection of TA clone sequencing

AxyPrepTM PCR product cleaning kit (purchased from AXYGEN) was used to purify the PCR product. Then the purified PCR product was added with sticky end by DNA A-Tailing Kit (purchased from TaKaRa). The product was linked to T vector (pMDTM19-T Vector Cloning Kit, purchased from TaKaRa) by a DNA Ligation Kit Ver2.1 (purchased from TaKaRa), and the ligated product was transformed into competent cells (DH5 alpha). Then, it was coated on an LB plate containing ampicillin resistance and cultured in 37 °C incubator for about 12 hours. Subsequently, a single colony was picked, and the single colony liquid was sent to Jin Weizhi for sequencing. Knockout efficiency = Mutatant Clone Number/Total Clone Number.

### (3) Cell counting by flow cytometry

10E5 to 10E8 cells were subject to centrifugation at 2000rpm for 5min. The supernatant was discarded, and 100 µl PBS buffer was added to each tube for resuspending the cells. Then, 5µl anti-human AB TCR-APC (purchased from eBioscience) antibody and 5µl HLA-A02 Monoclonal Antibody (BB7.2), APC, eBioscince^{™} (purchased from invitrogen) antibody were mixed well and incubated at room temperature for 10min. After centrifugation at 2000rpm for 5min, followed by washing with PBS buffer 2 times, the cells were resuspended and detected by using BD FACSAria flow cytometer to give the expression positive rates of the TCR and HLA-A02 on the cell surface. Knockout efficiency = (A-B)/A × 100%; A is the expression positive rate of the control group; and B is the expression positive rate of the knockout group.

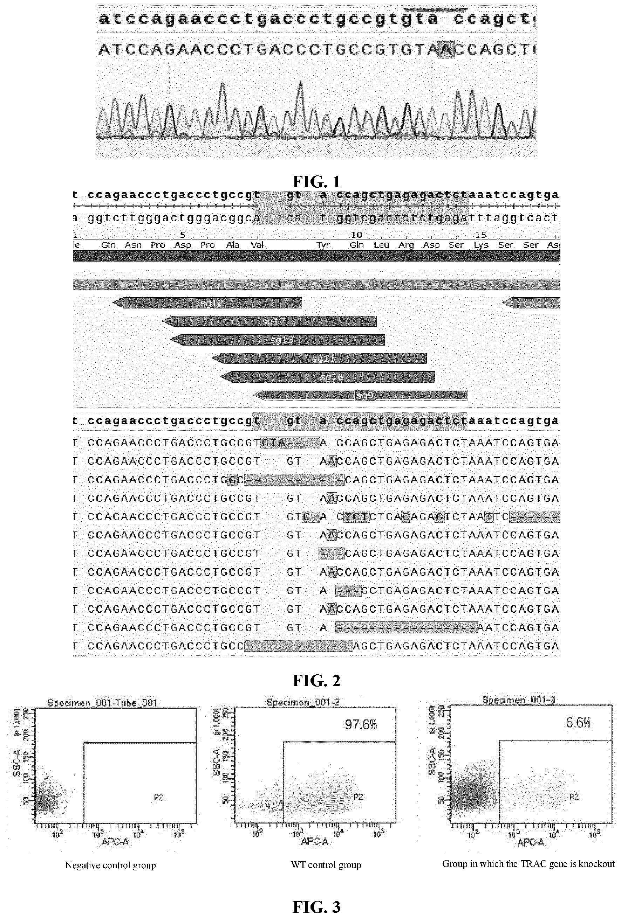

The three measured results of single knockout TRAC were shown in Figs. 1-3, and the calculated results of knockout efficiency were shown in Table 1. The results of three detection methods are substantially the same, and in the subsequent experiments, the editing efficiency was merely detected via Sanger sequencing.

**Table 1 Results of methods for detecting knockout efficiency of gene**

| Target gene | sgRNA | Detecting method | Knockout efficiency % |
|---|---|---|---|
| TRAC | Sg9 | Sanger sequencing | 90 |
| | | TA clone sequencing | 95 |
| | | Flow cytometry | 93 |

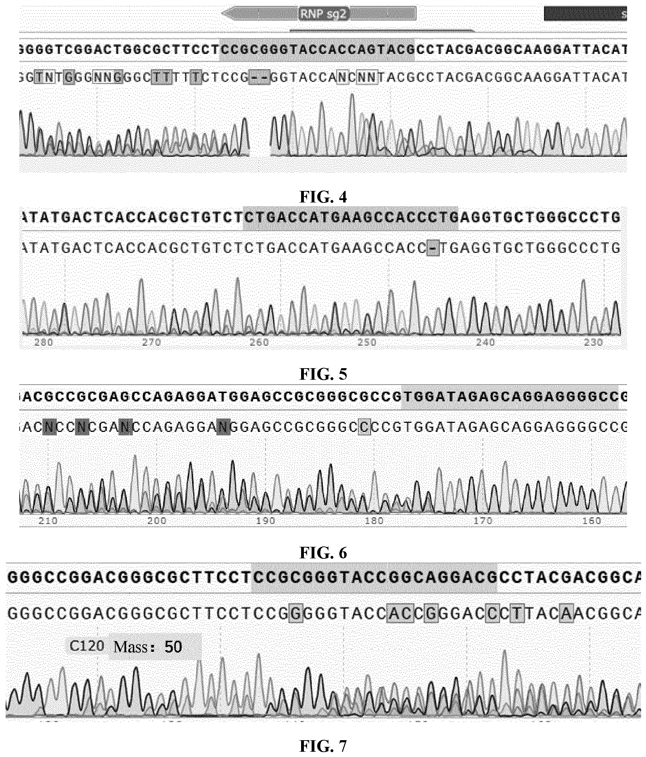

The results of Sanger sequencing against HLA-A02 gene editing were shown in FIGs. 4-5 with the editing efficiency of 90%; and the results of Sanger sequencing against HLA-A11 gene editing were shown in FIGs. 6-7.

### EXAMPLE 5 Preparation of T cells in which the TRAC gene and the HLA-A gene were dual knockout

RNP complexes were transferred into activated T cells prepared in Example 2 by electroporation using an electroporation kit (purchased from Lonza, Article No.:V4XXP-3024). The medium was preheated in the well plate for 30 min in advance (X-VIVO15 medium + 10% FBS + IL2 (200U/ml) + IL7 (10ng/ml) + IL15 (5ng/ml)). The electrokinetic buffer was formulated in a ratio of Nucleofector Solution: Supplement =82:18. Preparation of RNP complex: 20µg TRAC sgRNA (TRAC Sg9), 20µg HLA-A sgRNA (HLA-A02 Sg2 or HLA-A02 Sg5 or HLA-A11 sg21 or sgRNAs targeting HLA-A^{∗}24:02:01, HLA-A^{∗}30:01:01:01, HLA-A^{∗}33:01:01:01, HLA-A^{∗}03:01:01:01, HLA-A^{∗}01:01:01:01 or HLA-A^{∗}26:01:01:01) were added into PCRs (RNA-free), respectively, and 10µg Cas9 protein (purchased from thermo, Item No.A36499) was added to each tube. After mixing well gently, the mixtures were incubated at room temperature for 12min. The activated T cells cultured in Example 2 were counted, and centrifuged at 300g for 8min. The supernatant was discarded. PBS was added to resuspend cells. 1E7 cells were centrifuged at 300 g for additional 8 min. The supernatant was discarded, and the cells were resuspended in 100µl prepared electrofection buffer. The incubated RNP complexes of TRAC and HLA-A were added into the above cell suspensions and mixed gently. Then, the mixture was gently transferred to the electrofection cup. The electrofection cup was placed on the Lonza-4D electric rotating instrument, and the EO-115 electric rotating program was selected for electric rotating. Pre-heated medium was added into the electrofection cup, and then the cells were transferred into the preheated medium in the well plate with a supporting pipette, and then cultured in a 37 °C, 5% CO₂ incubator.

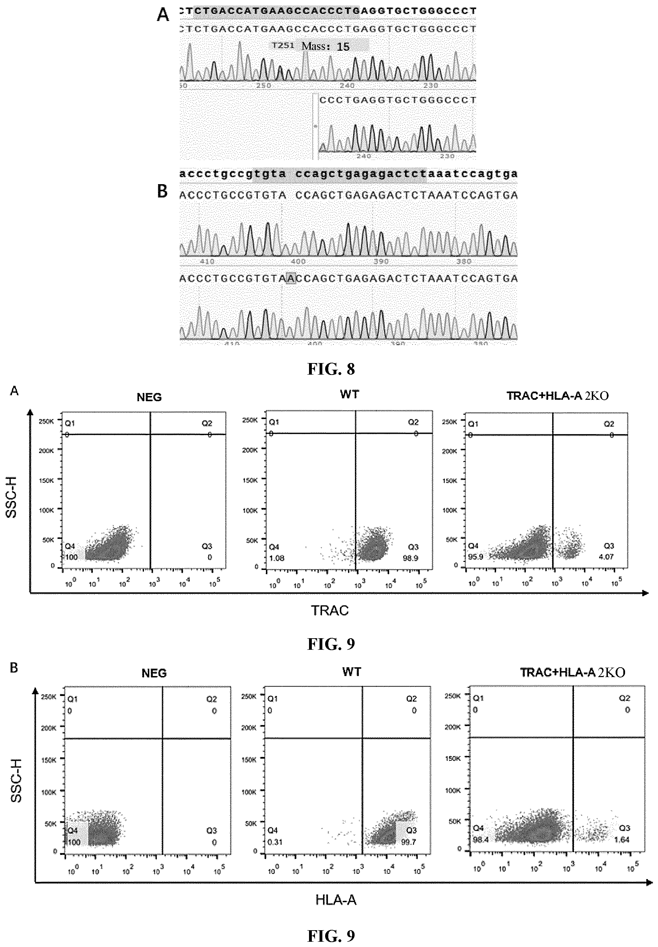

The dual knockout efficiency was detected via sequencing to obtain the TRACnegative, HLA-A-negative T cells in which the dual knockout efficiency was not less than 80%. The results were shown in FIGs. 8-9, wherein, FIG. 8A showed the results of knockout of HLA-A02 with HLA-A02 Sg5, wherein the upper row showed the results of the control group (that is, no knockout was performed with HLA-A02 Sg5); and the lower row showed the results of dual knockout of HLA-A02 and TRAC; wherein, FIG. 8B showed the results of knockout of TRAC with TRAC Sg9, wherein the upper row showed the results of the control group (that is, no knockout was performed with TRAC Sg9), and the lower row showed the results of dual knockout of HLA-A02 and TRAC. FIGs. 9A-9B showed the knockout status of HLA-A02 and TRAC in terms of protein levels, in which NEG referred to the negative control, WT referred to the status with no knockout, and TRAC+HLA-A dual knockout referred to the results of dual knockout of HLA-A02 and TRAC.

### EXAMPLE 6 Difference of the expression of TRAC gene, HLA-A gene, B2M gene and CIITA gene in dual knockout T cells from the expression of the corresponding genes in the corresponding cells

(1) The activated T cells prepared in Example 2 were divided into two groups: one was used as control, and the other was used to prepare T cells in which the TRAC gene and the HLA-A gene were dual knockout in accordance with the method of Example 5, and subject to Sanger sequencing in accordance with the method of step (1) of Example 4. In accordance with the sequencing results, the cells in which the TRAC gene and the HLA-A gene were dual knockout. The prepared dual knockout T cells were incubated with the corresponding TRAC and HLA-A antibodies, and sorted by flow cytometry or magnetic beads to give dual knockout cell strains.
(2) The changes of mRNA expression levels of dual knockout T cells and the control group were detected. An RNA extract kit (purchased from QIAGEN, Article No.: 74004) was used to extract the RNA. The RNA was subject to reverse transcription with a reverse transcription kit (purchased from Applied Biosystems, Article No.: 4368814) to give cDNA, which was used as a template for quantative PCR detection.
(3) The changes of protein expression levels of dual knockout T cells and the control group were detected. Total protein extract reagent (purchased from Thermo Scientific, Article No.: 87787) was used to extract protein. The expression levels of protein were detected by Western Blot method or flow cytometric method. The used antibodies were TRAC antibody (purchased from eBioscience, Article No.: 17-9986-42), HLA-A antibody (purchased from Merck, Article No.: 17-9876-41), B2M antibody (purchased from Invitrogen, Article No.: A15770) and CIITA antibody (purchased from OriGene, Article No.: CF812200), respectively.

The Sanger sequencing showed that in the dual knockout T cells, the nucleotide sequences of TRAC and/or HLA-A genes were changed as compared with the control group. The quantative PCR showed that in the dual knockout T cells, the expression levels of mRNA of TRAC and/or HLA-A genes were down-regulated, while the expression levels of mRNA of B2M and/or CIITA genes were not down-regulated. The results of FACS and Western Blot shown that in the dual knockout T cells, the expression levels of protein were down-regulated, while the expression levels of protein of B2M and/or CIITA were not down-regulated.

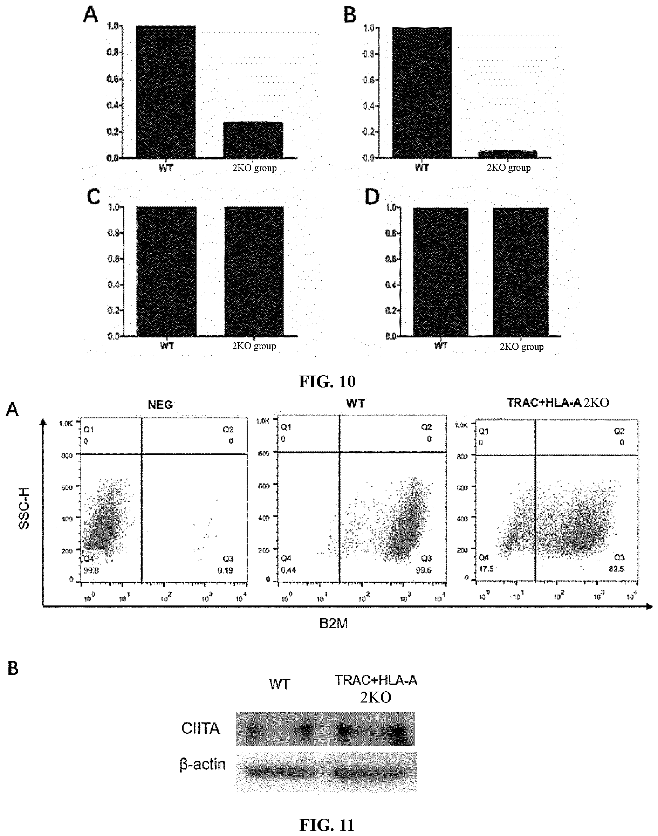

The results were shown in FIGs. 10-11, wherein, FIG. 10 showed the measurement of mRNA level of the gene expression, wherein FIGs. 10A-10D showed the mRNA levels of TRAC, HLA-A, B2M and CIITA, respectively; wherein WT referred to the status with no knockout, and the dual knockout group referred to the results of T cells in which the TRAC gene and the HLA-A gene were dual knockout. FIG. 11 showed the measurement of protein levels of the gene expression, wherein FIGs. 11A-11B showed the protein expression levels of B2M and CIITA, respectively; wherein NEG referred to the negative control, WT referred to the status with no knockout, and TRAC+HLA-A dual knockout referred to the results of T cells in which the TRAC gene and the HLA-A gene were dual knockout.

### EXAMPLE 7 Preparation of T cells in which the TRAC gene, the HLA-A/B2M gene and the CIITA gene were triple knockout, and verification of the change of expression of the corresponding three genes therein

(1) The control group and cells in which the TRAC gene, the HLA-A gene, and the CIITA gene were triple knockout, as well as cells in which the TRAC gene, the B2M gene, and the CIITA gene were triple knockout were prepared in accordance with the methods of step (1) of Example 6.
(2) The change of protein expression levels was detected in accordance with the method of step (3) of Example 6 by FACS and Western Blot methods.

In relation to the cells of the control group, the protein expression levels of TRAC, HLA-A and CIITA genes in T cells in which the TRAC gene, the HLA-A gene, and the CIITA gene were triple knockout were down-regulated. In relation to the cells of the control group, the protein expression levels of TRAC, HLA-A and CIITA genes in TRAC, B2M and CIITA triple knockout T cells were down-regulated.

(3) TRAC (purchased from eBioscience, Article No.: 17-9986-42), HLA-A (purchased from Merck, Article No.: 17-9876-41), B2M (purchased from Invitrogen, Article No.: A15770) antibodies were used to detect the knockout efficiency of the dual knockout cells of Example 6 and the two types of triple knockout cells in this example by flow cytometry. The results showed that in terms of the efficiency of multiple gene knockout at the single cell level, dual knockout was significantly better than triple knockout.

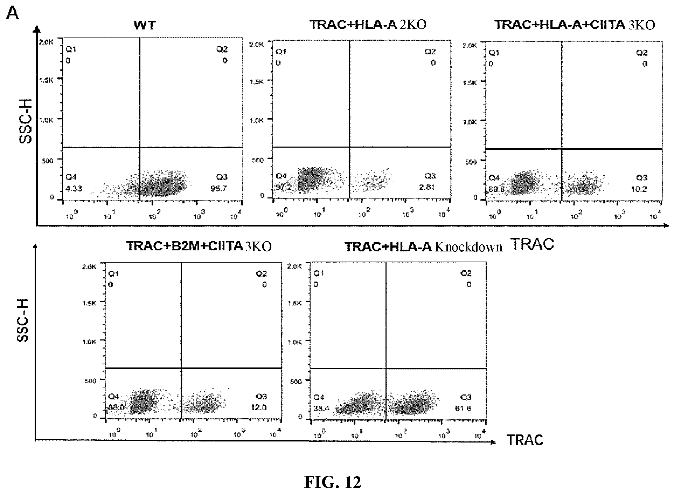

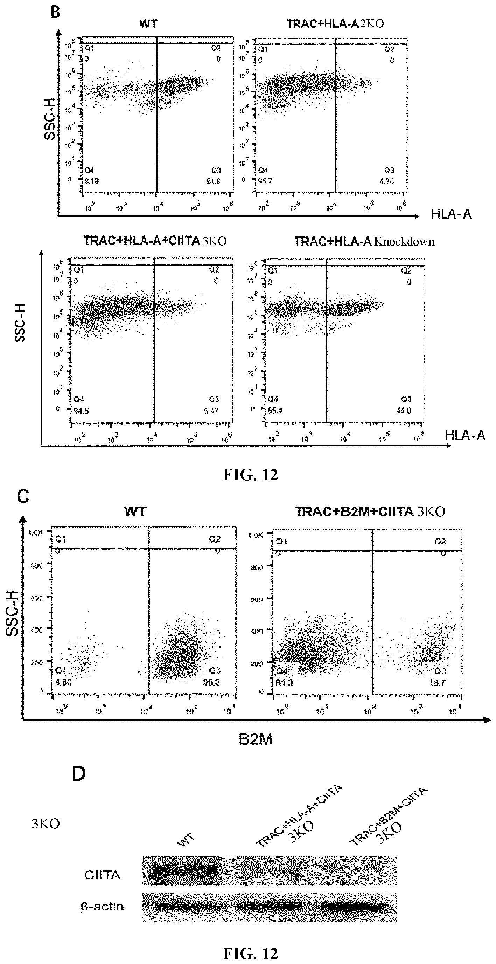

The results were shown in FIGs. 12A -12D, wherein FIGs. 12A-12C sequentially showed the knockout status of TRAC, HLA-A and B2M in terms of protein levels; wherein, WT referred to the status with no knockout, TRAC+HLA-A dual knockout referred to the results of T cells in which the TRAC gene and the HLA-A gene were dual knockout; TRAC+HLA-A+CIITA triple knockout referred to the results of T cells in which the TRAC gene, the HLA-A gene, and the CIITA gene were triple knockout; wherein TRAC+B2M+CIITA triple knockout referred to the results of T cells in which the B2M gene, the CIITA gene, and the TRAC gene were triple knockout; TRAC+HLA-A knockdown referred to the results of T cells in which the TRAC gene and the HLA-A gene prepared in Example 9 were knockdown. FIG. 12D showed the knockout status of CIITA in terms of protein level.

The results of FIG. 12 showed that as compared with the WT control group, the protein levels of TRAC, HLA-A, CIITA and B2M were down-regulated. At the same time, as compared with TRAC+HLA-A+CIITA triple knockout or TRAC+B2M+CIITA triple knockout, the knockout efficiency of TRAC+HLA-A dual knockout was higher.

### EXAMPLE 8 Design of antisense RNA sequence

Through the database https://www.ncbi.nlm.nih.gov/ or www.ensembl.org/, the transcription RNA sequences (as shown in SEQ ID NO. 82-90) of the corresponding genes (TRAC gene and HLA-A gene) were obtained, and siRNA was designed by reference to the following principles:

Sequences of 50-100 nucleotides downstream of the start codon and 100 nucleotides upstream of the stop codon were avoided as possible. Sequences with less than 30 nucleotides in length were selected. Four or greater consecutive identical bases were avoided. Intron regions were avoided. Repetitive sequences were avoided. Single nucleotide polymorphism (SNP) sites were avoided. Sequences have a GC content ranging 30% to 60%. Sequence patterns AA (N < sub > 19), NA (N < sub > 21) or NAR (N < sub > 17) YNN were preferentially selected, wherein A was adenylate; T was thymosine; R was adenylate or guanosine (purine); Y was thymidine or cytidine (pyrimidine); N was adenylate, thymidine, guanosine or cytidine. The selected sequences were subject to comparison and analysis of sequence homology, so as to avoid the significant homology between antisense RNA and other genes or sequences and the resulting off-target effect. The homology analysis was performed by means of NCBI Blast tool: Nucleotide-nucleotide BLAST(blastn), UCSC Blat tool or Ensembl Blast.

The designed antisense RNA sequence included: HLA-A-homo-551 (which included the nucleotide sequence as shown in SEQ ID NO. 93); HLA-A-homo-NEG (which included the nucleotide sequence as shown in SEQ ID NO. 94); TRAC-homo-375 (which included the nucleotide sequence as shown in SEQ ID NO. 95); and TRAC-homo-NEG (which included the nucleotide sequence as shown in SEQ ID NO. 96).

### EXAMPLE 9 Preparation of T cells in which the TRAC gene and the HLA-A gene were knockdown

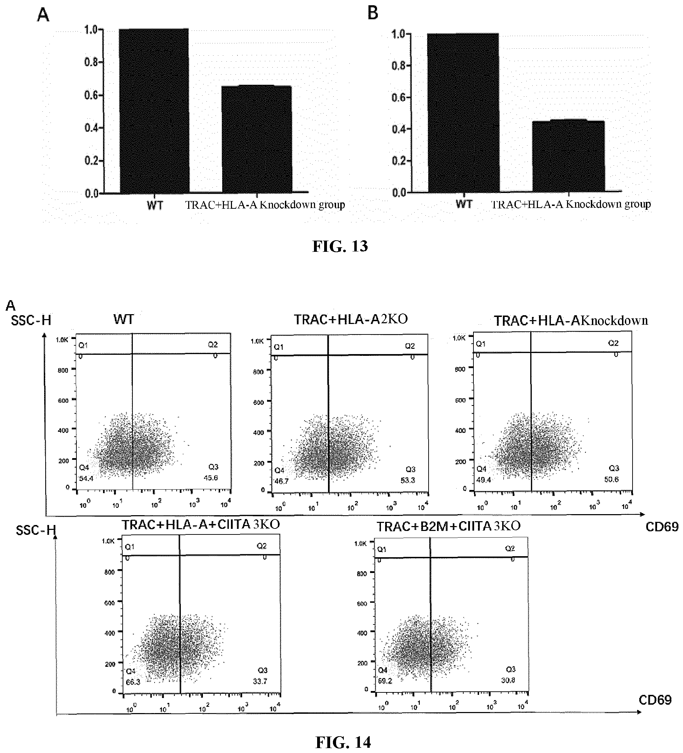

The antisense RNAs designed in Example 8 were used to perform double gene knockdown. A lentivirus including the antisense RNA sequences of TRAC gene and HLA-A gene was prepared (Gemma). CD3⁺ T cells were prepared in accordance with the method of Example 2 (Day D0), and activated with CD3/CD28 antibody-bearing magnetic beads. The activated T cells were transfected with the lentiviruses bearing the antisense RNA sequences of TRAC gene and HLA-A gene (SEQ ID NO. 95 and SEQ ID NO. 93) (Day D1), washed to remove the lentivirus vectors on Day D2, and continued to culture until Day D5. The cultured T cells cultured to Day D5 were collected, and detected by quantative PCR or Western Blot and the like for the knockdown efficiency. The resultant T cells were labelled with the corresponding TRAC and HLA-A antibodies, and sorted by flow cytometric sorting or magnetic bead sorting to give T cells in which the TRAC gene and the HLA-A gene were knockdown. The results showed that in the TRAC and HLA-A gene knockdown group, the expression levels of mRNA and protein of TRAC and HLA-A were both down-regulated, wherein, FIGs. 13A-13B sequentially showed the knockout status of TRAC and HLA-A in terms of mRNA levels; wherein, WT referred to the status with no knockout, TRAC+HLA-A dual knockout referred to the results of T cells in which the TRAC gene and the HLA-A gene were dual knockout. Of those, for the knockout level of TRAC and HLA-A in terms of protein levels, please refer to the results of FIG. 12.

### EXAMPLE 10 Difference of activities of different T cells

T cells in which no gene was knockout, 2 or 3 genes were knockout, and 2 genes were knockdown in Examples 2, 5, 7, and 9 were prepared, and compared for their activities. The cells of individual group were counted, and 1^{∗}10⁶ cells were inoculated into a 24-well plate. In each well, PHA(0.3µg/ml) (ionomycin+) or 5ng/ml PMA and 50 ng/ml ionomycin were added into the cells, which were cultured for additional 5 hours, and then detected by CD69 (early activated) (purchased from BD Biosciences, Article No.: FN50), CD137 (later) (purchased from BD Biosciences, Article No.: 4B4-1) antibodies via flow cytometry for the activated status of the cells. The results showed that the activities of dual knockout and 2 gene knockdown T cells were better than triple knockout cells.

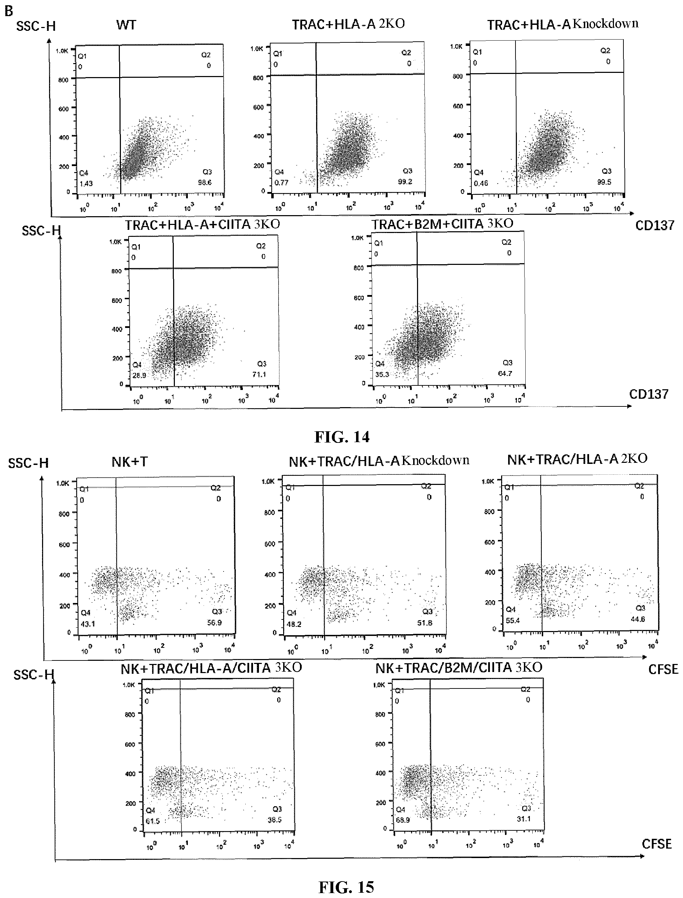

The expression status of CD69 and CD137 in terms of protein levels were shown in FIGs. 14A-14B, respectively, wherein, WT referred to the status with no knockout, TRAC+HLA-A dual knockout referred to the results of T cells in which the TRAC gene and the HLA-A gene were dual knockout; TRAC+HLA-A+CIITA triple knockout referred to the results of T cells in which the TRAC gene, the HLA-A gene, and the CIITA gene were triple knockout; wherein TRAC+B2M+CIITA triple knockout referred to the results of T cells in which the B2M gene, the CIITA gene, and the TRAC gene were triple knockout; and TRAC+HLA-A knockdown referred to the results of T cells in which the TRAC gene and the HLA-A gene prepared in Example 9 were knockdown.

### EXAMPLE 11 Difference of reactivity of different T cells on allogenic NK cells

T cells in which no gene was knockout, 2 or 3 genes were knockout, and 2 genes were knockdown in Examples 2, 5, 7 and 9 were labelled with CFSE (invitrogen, C34554). The cells were counted, and 1^{∗}10⁶ cells of each group were co-cultured with NK cells (NK92MI) at a ratio of 1:1, respectively. After 24 hours, the co-cultured cells were collected, and detected via flow cytometry for the ratio of CFSE positive cells in the mixed cells.

The results showed that the killing toxicity of NK cells on the dual gene knockout, dual knockdown T cells was lower than that on triple knockout T cells. The results were shown in FIG. 15, wherein, NK+T referred to the status that the NK cells were co-cultured with T cells with no knockout; NK+ TRAC+HLA-A knockdown referred to the status that the NK cells were co-cultured with T cells in which the TRAC gene and the HLA-A gene prepared in Example 9 were knockdown; NK+TRAC+HLA-A dual gene knockout referred to the status that the NK cells were co-cultured with T cells in which the TRAC gene and the HLA-A gene were dual knockout; NK+ TRAC+HLA-A+CIITA triple gene knockout referred to the status that the NK cells were co-cultured with T cells in which the TRAC gene, the HLA-A gene, and the CIITA gene were triple knockout; and NK+ TRAC+B2M+CIITA triple gene knockout referred to the status that the NK cells were co-cultured with T cells in which the B2M gene, the CIITA gene, and the TRAC gene were triple knockout.

### EXAMPLE 12 Difference of allogenic rejections of different T cells

Peripheral blood originated from donor 1 was used to prepare T cells in which no gene was knockout, 2 or 3 genes were knockout, and 2 genes were knockdown in Examples 2, 5, 7 and 9. Peripheral blood originated from donor 2 was used to prepare CD3⁺T cells. Various groups of cells prepared with peripheral blood from donor 1 were mixed with CD3⁺ T cells prepared with peripheral blood from donor 2 in accordance with Example 2 at a ratio of 1:1. After 24 hours, the mixed system of cells was detected for the expression level of IFN-γ. The results showed that the expression level of IFN-γ in dual knockout T cell group was lower than that in triple knockout T cell group.

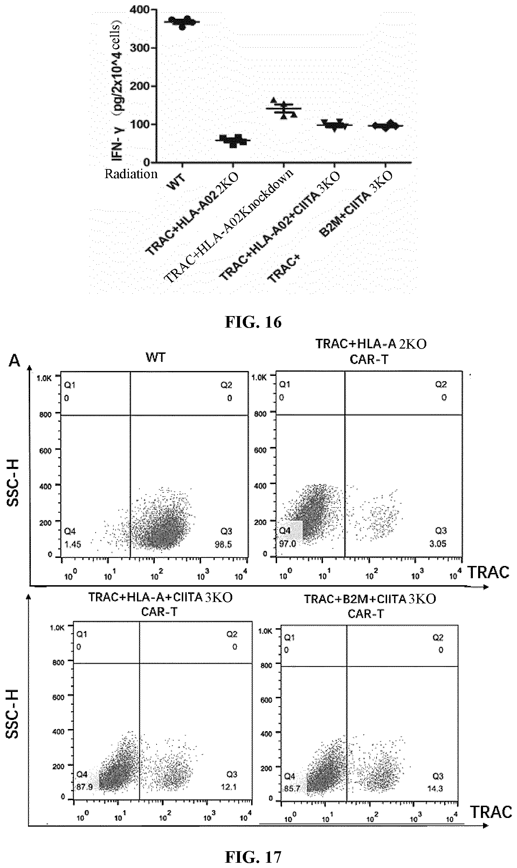

The results are shown in FIG. 16, wherein WT referred to the status with no knockout; TRAC+HLA-A dual knockout referred to the results of T cells in which the TRAC gene and the HLA-A gene were dual knockout ; TRAC+HLA-A+CIITA triple knockout referred to the results of T cells in which the TRAC gene, the HLA-A gene and the CIITA gene were triple knockout; wherein TRAC+B2M+CIITA triple knockout referred to the results of T cells in which the B2M gene, the HLA-A gene and the TRAC gene were triple knockout; and TRAC+HLA-A knockdown referred to the results of T cells in which the TRAC gene and the HLA-A gene prepared in accordance with Example 9 were knockdown.

### EXAMPLE 13 Preparation of CAR-T cells in which the TRAC gene and the HLA-A gene were dual knockout, CAR-T cells in which the TRAC gene, the HLA-A gene, and the CIITA gene were triple knockout, and CAR-T cells in which the TRAC gene, the B2M gene and the CIITA gene were knockout

(1) CD3⁺ T cells were prepared in accordance with the method of Example 2 (Day D0), and activated with CD3/CD28 antibody-bearing magnetic beads. The activated T cells were transfected with lentiviruses (lentiviruses containing CD19-CAR, CD20-CAR or BCMA-CAR, etc.) on D1, washed to remove the lentivirus vectors on D2, sorted for CAR-positive T cells at D3, and continued to culture until Day D5.
(2) The CAR-T cells on D5 were selected as initial cells, and used to prepare cells in which the TRAC gene and the HLA-A gene were dual knockout, CAR-T cells in which the TRAC gene, the HLA-A gene and the CIITA gene were triple knockout, and CAR-T cells in which the TRAC gene, the B2M gene and the CIITA gene were triple knockout in accordance with the methods of Example 5 and Example 7, respectively.
(3) By detection via flow cytometry, dual knockout and triple knockout CAR-T cells were obtained, wherein the yield of dual knockout CAR-T cells were higher than that of triple knockout CAR-T cells.

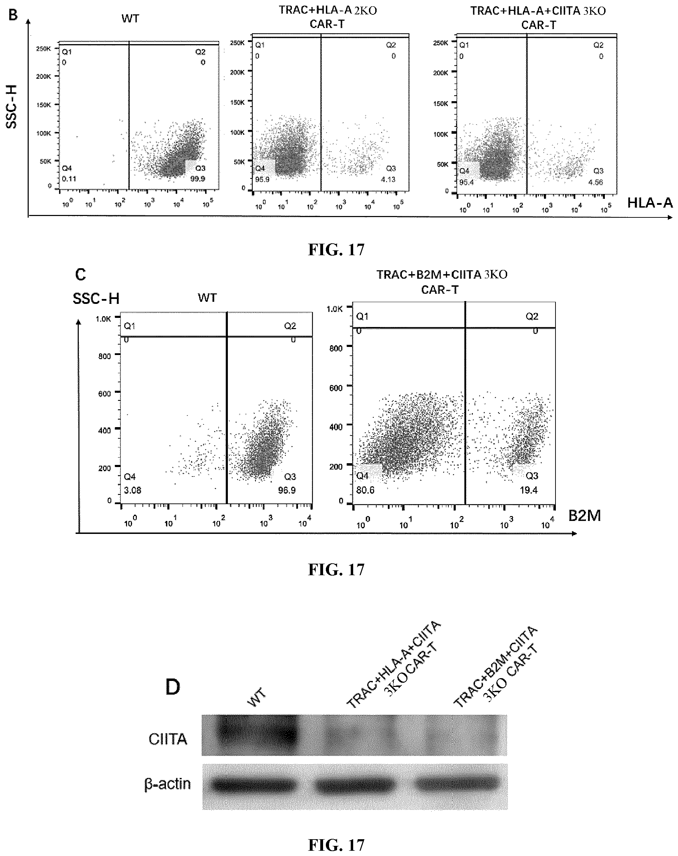

The results were shown in FIGs. 17A -17D, wherein, FIGs. 17A-17C sequentially showed the knockout status of TRAC, HLA-A and B2M in terms of protein levels; and FIG. 17D showed the knockout status of CIITA in terms of protein level, wherein, WT referred to the status with no knockout, TRAC+HLA-A dual knockout referred to the results of CAR-T cells in which the TRAC gene and the HLA-A gene were dual knockout; TRAC+HLA-A+CIITA triple knockout referred to the results of CAR-T cells in which the TRAC gene, the HLA-A gene and the CIITA gene were triple knockout; wherein TRAC+B2M+CIITA triple knockout referred to the results of CAR-T cells in which the B2M gene, the CIITA gene and the TRAC gene were triple knockout.

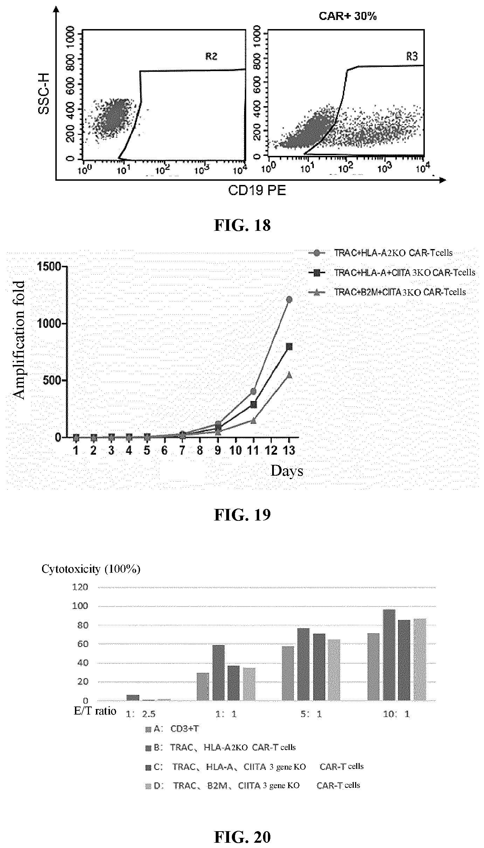

Of those, the transfection efficiency of CD19CAR was shown in FIGs. 18A-18B, wherein, CAR30%+ represented the transfection efficiency of CD19 CAR.

FIG. 19 showed the amplification folds of different cells. Of those, the CAR-T cells in which the TRAC gene and the HLA-A gene were dual knockout had the maximum amplification fold.

### EXAMPLE 14 Anti-tumor effect of CAR-T cells in which the TRAC gene and the HLA-A gene were dual knockout

CAR-T cells in which the TRAC gene and the HLA-A gene were dual knockout (targeting CD19, CD20 or BCMA) were prepared in Example 14. Target cells expressing luciferase gene (target gene-positive leukemia or lymphoma cell lines, such as, Raji, Jurkat, MM1S and the like) were inoculated into a well place. Then, double knockout CAR-T cells, triple knockout CAR-T cells, and zero knockout CAR-T cells were added at different ratios of efficiency to target(1 : 2.5, 1 : 1, 5: 1, 10: 1), respectively. After 24 hours of co-culture, the cells were transferred to the detection well plate. The luciferase substrate was added, and the fluorescence value was detected by a microplate reader. Killing efficiency = 1 - the fluorescence value of T cells co-cultured with target cells/the fluorescence value of target cells cultured alone.

The results showed that the CAR-T cells in which the TRAC gene and the HLA-A gene were dual knockout exhibit significant killing effect on tumor cells.

FIG. 20 showed that the killing effect on CD19 target cell Raji-Luciferase, wherein the CAR-T cells in which the TRAC gene and the HLA-A gene were dual knockout exhibit the most significant killing effect, wherein at each E/T ratio, the results corresponding to Notes A-D were shown from left to right.

### EXAMPLE 15 Anti-tumor effect of CAR-T cells in which the TRAC gene and the HLA-A gene were dual knockout

NSG mice were injected with tumor cells intravenously. After the tumor was successfully established, CAR-T cells in which the TRAC gene and the HLA-A gene were dual knockout, triplel knockout CAR-T cells, and CAR-T cells in which no gene was knockout were refused back to the mice. The tumor size was monitored in the mice.

Mice to which the double knockout CAR-T cells were refused exhibited significantly slower growth rate of tumor.

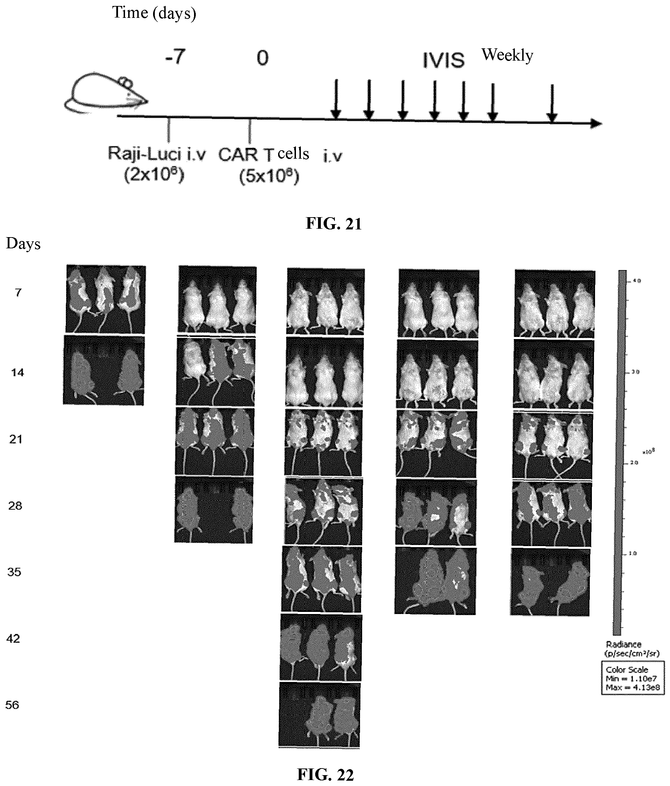

The results were shown in FIGs. 21-22, wherein, FIG. 21 showed the administration mode of mice, i.v. represented intravenous injection, CAR-T cells represented double knockout and triple knockout CAR-T cells expressing CD19 CAR. FIG. 20 showed the tumor size in mice after the mice were administered with CAR-T cells, wherein, FIG. 20 showed, from left column to right column, the tumor size in mice which were administered with normal saline, unmodified T cells, CD19 CAR-T cells in which the TRAC gene and the HLA-A gene were dual knockout, CD19 CAR-T cells in which TRAC, HLA-A and CIITA were triple knockout, CD19 CAR-T cells in which B2M, CIITA and TRAC were triple knockout in sequence. The results showed that the rate of tumor growth was significantly reduced in the mice re-infused with CAR-T cells in which both the TRAC gene and the HLA-A gene were knockout.

The aforesaid detailed description is provided in an illustrative and exemplary manner, and is not intended to limit the scope of the appended claims. Various modifications of embodiments currently listed in the present application are apparent for persons skilled in the art, and encompassed within the scope of the appended claims and their equivalents.

## Claims

1. A modified immunologic effector cell, wherein the expression and/or activity of a TRAC gene and a HLA-A gene are/is down-regulated, the expression and/or activity of a B2M gene are/is not down-regulated, and the expression and/or activity of a CIITA gene are/is not down-regulated, as compared with the expression and/or activity of the corresponding gene in the corresponding cell which is not modified.

2. The immunologic effector cell according to claim 1, wherein said modification results in down-regulation of expression and/or activity of two genes, and wherein the two genes consist of said TRAC gene and said HLA-A gene.

3. The immunologic effector cell according to any one of claims 1-2, wherein the expression and/or activity of said TRAC gene and said HLA-A gene are/is down-regulated, the expression and/or activity of said B2M gene are/is not down-regulated, and the expression and/or activity of said CIITA gene are/is not down-regulated, as compared with the corresponding wild type cell.

4. The immunologic effector cells according to any one of claims 1-3, wherein, as compared with the corresponding wild type cell, the expression and/or activity of the two genes are/is down-regulated, wherein said two genes consist of the TRAC gene and the HLA-A gene.

5. The immunologic effector cell according to any one of claims 1-4, wherein said immunologic effector cell comprises T cell.

6. The immunologic effector cell according to any one of claims 1-5, wherein the down-regulation of the expression level and/or activity of said genes comprise a down-regulation of expression and/or activity of a nucleic acid molecule encoding said genes; and/or a down-regulation of expression and/or activity of a protein product encoded by said genes.

7. The immunologic effector cell according to any one of claims 1-6, wherein said modification comprises gene mutation and/or gene silencing.

8. The immunologic effector cell according to any one of claims 1-7, wherein said modification comprises administering one or more substances selected from the group consisting of antisense RNA, siRNA, shRNA, and CRISPR/Cas9 system to said immunologic effector cell.

9. The immunologic effector cell according to any one of claims 1-8, wherein said modification comprises administering CRISPR/Cas9 system to said immunologic effector cell.

10. The immunologic effector cell according to any one of claims 1-9, wherein said modification comprises administering an sgRNA targeting an exon of said HLA-A gene to said immunologic effector cell.

11. The immunologic effector cell according to any one of claims 1-10, wherein said modification further comprises administering an sgRNA targeting an exon of said TRAC gene to said immunologic effector cell.

12. The immunologic effector cell according to claim 10, wherein said sgRNA targeting the exon of said HLA-A gene comprises a nucleotide sequence shown in any one of SEQ ID No. 16-54 and 91-92.

13. The immunologic effector cell according to claim 12, wherein said sgRNA targeting the exon of said TRAC gene comprises a nucleotide sequence shown in any one of SEQ ID No. 1-15.

14. The immunologic effector cell according to any one of claims 8-13, wherein said antisense RNA comprises a nucleotide sequence as shown in any one of SEQ ID No. 93-96.

15. The immunologic effector cell according to any one of claims 1-14, wherein said modification comprises administering a Cas enzyme to said cell.

16. The immunologic effector cell according to claim 15, wherein the Cas enzyme comprises a Cas9 protein.

17. The immunologic effector cell according to any one of claims 1-16, wherein said immunologic effector cell comprises a nucleic acid encoding a chimeric antigen receptor (CAR), and said CAR comprises an antigen binding domain, a hinge region, a transmembrane domain, a costimulatory structure, and a primary signal transduction domain.

18. The immunologic effector cell according to claim 17, wherein said antigen binding domain specifically binds to a tumor antigen.

19. The immunologic effector cell according to claim 17, wherein said tumor antigen is selected from the group consisting of CD19, CD20, and BCMA.

20. The immunologic effector cell according to any one of claims 17-19, wherein said antigen binding domain is selected from the group consisting of a monoclonal antibody, a polyclonal antibody, a human antibody, a humanized antibody, a single domain antibody, and an antigen binding fragment.

21. The immunologic effector cell according to any one of claims 17-20, wherein said antigen binding domain targets a solid tumor.

22. The immunologic effector cell according to claim 21, wherein said solid tumor is selected from the group consisting of liver cancer, stomach cancer, lung cancer, breast cancer, and non-small cell lung cancer.

23. The immunologic effector cell according to any one of claims 17-20, wherein said antigen binding domain targets a non-solid tumor.

24. The immunologic effector cell according to claim 23, wherein said non-solid tumor is selected from the group consisting of B cell lymphoma, Hodgkin's lymphoma, chronic myeloid leukemia, and acute myeloid leukemia.

25. The immunologic effector cell according to any one of claims 17-24, wherein said transmembrane domain comprises a transmembrane domain derived from a protein selected from the group consisting of CD28, CD3e, CD27, CD3ε and CD45.

26. The immunologic effector cell according to any one of claims 17-25, wherein said costimulatory domain comprises a costimulatory domain of protein selected from the group consisting of CD137, CD28, CD27, OX40 and CD30.

27. The immunologic effector cell according to any one of claims 17-26, wherein said hinge region connects said antigen binding domain with said transmembrane domain, and comprises a hinge region of protein selected from the group consisting of human immunoglobulin hinge region, a GS linker, a KIR2DS2 hinge region, and a CD8a hinge region.

28. A method of preparing said modified immunologic effector cell according to any one of claims 1-27, comprising the following steps: down-regulating the expression and/or activity of said TRAC gene and said HLA-A gene, not down-regulating the expression and/or activity of said B2M gene, and not down-regulating the expression and/or activity of said CIITA gene, as compared with the expression and/or activity of said corresponding gene in said corresponding cell which is not modified.

29. The method according to claim 28, wherein said modification results in down-regulation of expression and/or activity of two genes, and wherein said two genes consist of the TRAC gene and the HLA-A gene.

30. The method according to any one of claims 28-29, wherein the expression and/or activity of said TRAC gene and said HLA-A gene are/is down-regulated, the expression and/or activity of said B2M gene are/is not down-regulated, and the expression and/or activity of said CIITA gene are/is not down-regulated, as compared with the corresponding wild type cell.

31. The method according to any one of claims 28-30, wherein, as compared with the corresponding wild type cell, the expression and/or activity of the two genes are/is down-regulated, and wherein said two genes consist of the TRAC gene and the HLA-A gene.

32. The method according to any one of claims 28-31, wherein the down-regulation of the expression level and/or activity of said genes comprise a down-regulation of expression and/or activity of a nucleic acid molecule encoding said genes; and/or a down-regulation of expression and/or activity of a protein product encoded by said genes.

33. The method according to any one of claims 28-32, wherein said modification comprises gene mutation and/or gene silencing.

34. The method according to any one of claims 28-33, wherein said modification comprises administering one or more substances selected from the group consisting of antisense RNA, siRNA, shRNA, and CRISPR/Cas9 system to said immunologic effector cell.

35. The method according to any one of claims 28-34, wherein said modification comprises administering CRISPR/Cas9 system to said immunologic effector cell.

36. The method according to any one of claims 28-35, wherein said modification comprises administering an sgRNA targeting an exon of said HLA-A gene to said immunologic effector cell.

37. The method according to any one of claims 28-36, wherein said modification comprises administering an sgRNA targeting an exon of said TRAC gene to said immunologic effector cell.

38. The method according to claim 36, wherein the sgRNA targeting the exon of said HLA-A gene comprises a nucleotide sequence shown in any one of SEQ ID No. 16-54 and 91-92.

39. The method according to claim 37, wherein the sgRNA targeting the exon of said TRAC gene comprises a nucleotide sequence shown in any one of SEQ ID No. 1-15.

40. The method according to any one of claims 34-39, wherein said antisense RNA comprises a nucleotide sequence as shown in any one of SEQ ID No. 93-96.

41. The method according to any one of claims 34-40, wherein said modification further comprises administering a Cas enzyme to said cell.

42. The method according to claim 41, wherein the Cas enzyme comprises a Cas9 protein.

43. A composition, comprising said immunologic effector cell according to any one of claims 1-27 and a pharmaceutically acceptable carrier.

44. The composition according to claim 43, comprising a cell population which comprises said immunologic effector cell according to any one of claims 1-27.

45. Use of said immunologic effector cell according to any one of claims 1-27 for preparing a CAR-T cell.

46. Use of said immunologic effector cell according to any one of claims 1-27 in manufacture of a drug for allogeneic therapy.

47. Use of said immunologic effector cell according to any one of claims 1-27 in manufacture of a drug for treating a tumor.

48. The use according to claim 47, wherein said tumor comprises solid and non-solid tumors.

49. The use according to any one of claims 47-48, wherein said tumor is selected from the group consisting of liver cancer, stomach cancer, lung cancer, breast cancer, non-small cell lung cells, B cell lymphoma, Hodgkin's lymphoma, chronic myeloid leukemia, and acute myeloid leukemia.
